# EUROPEAN PATENT APPLICATION

(11) **EP 2 153 840 A1**
(43) Date of publication of application: **17.02.2010**
(21) Application number: 08752845.1
(22) Date of filing: 09.05.2008
(51) Int. Cl.: A61K 38/00, A61K 39/395, A61K 45/00, A61P 9/00, A61P 9/10, A61P 13/12, G01N 33/53, C07K 14/47, C07K 16/18

(54) **THERAPEUTIC AGENT AND DETECTION REAGENT FOR ARTERIOSCLEROTIC DISEASE WHICH TARGETS FOR THALLUSIN**

(30) Priority: 11.05.2007 JP 2007127131
(71) Applicant: Proteinexpress Co., Ltd., Chuo-ku Chiba-shi Chiba 260-0856 (JP); Showa University, Tokyo, 1428555 (JP); National University Corporation Tokyo Medical and Dental University, Tokyo 113-8510 (JP)
(72) Inventor: WATANABE, Takuya, Tokyo 151-0061 (JP); SHICHIRI, Masayoshi, Tokyo 174-0065 (JP)
(74) Representative: Tollervey, Rebecca Marie
(86) International application number: PCT/JP2008/058992
(87) International publication number: WO 2008/143151

(57) **Abstract**

The present invention provides a prophylactic and therapeutic agent for arteriosclerotic diseases and a method or reagent for detecting arteriosclerotic diseases. Further, the present invention provides a therapeutic agent for an arteriosclerotic disease, comprising salusin-α as an active ingredient, a therapeutic agent for an arteriosclerotic disease, comprising an antagonist of salusin-β as an active ingredient, and a method for detecting an arteriosclerotic disease, comprising assaying salusin-α in a biological sample.

SEQUENCE LISTING

<110> ProteinExpress Co. Ltd.
<110> Showa University
<110> National University Corporation Tokyo Medical and Dental University

<120> A therapeutic agent and detecting agent of arteriosclerotic
      diseases which target salusins

<130> PH-3550-PCT

<160> 8

<170> PatentIn version 3.4

<210> 1
<211> 28
<212> PRT
<213> Homo sapiens

<400> 1

Ser Gly Ala Leu Pro Pro Ala Pro Ala Ala Pro Arg Pro Ala Leu Arg
1               5                   10                  15

Ala Gln Arg Ala Gly Pro Ala Gly Pro Gly Ala Lys
            20                  25

<210> 2
<211> 29
<212> PRT
<213> Homo sapiens

<400> 2

Ser Gly Ala Leu Pro Pro Ala Pro Ala Ala Pro Arg Pro Ala Leu Arg
1               5                   10                  15

Ala Gln Arg Ala Gly Pro Ala Gly Pro Gly Ala Lys Gly
            20                  25

<210> 3
<211> 20
<212> PRT
<213> Homo sapiens

<400> 3

Ala Ile Phe Ile Phe Ile Arg Trp Leu Leu Lys Leu Gly His His Gly
1               5                   10                  15

Arg Ala Pro Pro
            20

<210> 4
<211> 242
<212> PRT
<213> Homo sapiens

<400> 4

Met Ala Ala Ala Thr Arg Gly Cys Arg Pro Trp Gly Ser Leu Leu Gly
1               5                   10                  15

Leu Leu Gly Leu Val Ser Ala Ala Ala Ala Ala Trp Asp Leu Ala Ser
            20                  25                  30

Leu Arg Cys Thr Leu Gly Ala Phe Cys Glu Cys Asp Phe Arg Pro Asp
        35                  40                  45

Leu Pro Gly Leu Glu Cys Asp Leu Ala Gln His Leu Ala Gly Gln His
    50                  55                  60

Leu Ala Lys Ala Leu Val Val Lys Ala Leu Lys Ala Phe Val Arg Asp
65                  70                  75                  80

Pro Ala Pro Thr Lys Pro Leu Val Leu Ser Leu His Gly Trp Thr Gly

                85                  90                  95

Thr Gly Lys Ser Tyr Val Ser Ser Leu Leu Ala His Tyr Leu Phe Gln
            100                 105                 110

Gly Gly Leu Arg Ser Pro Arg Val His His Phe Ser Pro Val Leu His
        115                 120                 125

Phe Pro His Pro Ser His Ile Glu Arg Tyr Lys Lys Asp Leu Lys Ser
    130                 135                 140

Trp Val Gln Gly Asn Leu Thr Ala Cys Gly Arg Ser Leu Phe Leu Phe
145                 150                 155                 160

Asp Glu Met Asp Lys Met Pro Pro Gly Leu Met Glu Val Leu Arg Pro
                165                 170                 175

Phe Leu Gly Ser Ser Trp Val Val Tyr Gly Thr Asn Tyr Arg Lys Ala
            180                 185                 190

Ile Phe Ile Phe Ile Arg Trp Leu Leu Lys Leu Gly His His Gly Arg
        195                 200                 205

Ala Pro Pro Arg Arg Ser Gly Ala Leu Pro Pro Ala Pro Ala Ala Pro
    210                 215                 220

Arg Pro Ala Leu Arg Ala Gln Arg Ala Gly Pro Ala Gly Pro Gly Ala
225                 230                 235                 240

Lys Gly

<210> 5

<211>  18
<212>  DNA
<213>  Artificial

<220>
<223>  primer

<400>  5
cctggcaccc agcacaat                                                    18


<210>  6
<211>  18
<212>  DNA
<213>  Artificial

<220>
<223>  primer

<400>  6
gggccggact cgtcatac                                                    18


<210>  7
<211>  25
<212>  DNA
<213>  Artificial

<220>
<223>  primer

<400>  7
ttcggaatat catcaaacag gagcc                                            25


<210>  8
<211>  21
<212>  DNA
<213>  Artificial

<220>
<223>  primer

<400>  8

cacacctggc aagatggagt t 21

## Description

### Technical Field

The present invention relates to a prophylactic and therapeutic agent for arteriosclerotic diseases, targeting salusin-α or salusin-β. The present invention further relates to a method or reagent for detecting arteriosclerotic diseases by targeting salusin-α.

### Background Art

According to the 2005 "Dynamic Statistics of the Population" published by the Ministry of Health, Labor and Welfare, the death toll from cancer in Japan was 326,000, making it the primary cause of death, followed by cardiac disease (173,000), which is the secondary cause of death, and stroke (133,000), which is the tertiary cause of death. The total death toll from cardiac disease and stroke is almost equivalent to that from cancer. In particular, ischemic heart disease, which is a representative example of cardiac disease, and brain infarction, which is a typical disease pattern of stroke, are developed with arteriosclerosis as basic pathological conditions. Therefore, prevention of the onset and development of arteriosclerosis is significant for maintaining and enhancing the nation's health.

A characteristic pathologic finding in primary lesions of arteriosclerosis is aggregation of macrophage-derived foam cells wherein cholesterol ester is accumulated within cells. Macrophages incorporate modified LDLs, such as oxidized low density lipoprotein (oxidized LDL), via various scavenger receptors into the cells. Cholesterol ester that is a major cholesterol of oxidized LDL is degraded to free cholesterol and fatty acid in lysosomes. When the intracellular free cholesterol level reaches an excessive degree, it is regulated by 2 mechanisms. The first mechanism is cholesterol efflux by ATP-binding cassette transporter A1 (ABCA-1) to the outside of the cells, and the second mechanism is conversion to cholesterol ester by acyl-CoA: cholesterol acyltransferase-1 (ACAT-1). When cholesterol ester is accumulated in the cytoplasm, macrophages are converted into foam cells, forming primary lesions of arteriosclerosis.

Another characteristic pathologic finding in primary lesions of arteriosclerosis is aggregation of macrophage-derived foam cells in which cholesterol ester is accumulated. Macrophage-derived foam cells secrete various cytokines, inducing migration of vascular smooth muscle cells from the tunica media of an artery to the arterial intima or proliferation thereof in the arterial intima. Vascular smooth muscle cells that have migrated to the arterial intima synthesize and secrete an extracellular matrix, thickening the arterial intima. Such intimal thickening causes vascular lumen narrowing and reduced blood flow. Foam cells from vascular smooth muscle cells are also found at and after mid-stage arteriosclerosis.

Salusins are circulation-regulating substances recently identified by bioinformatics analyses. There are two related peptides, designated salusin-α and salusin-β (see Non-Patent Document 1). Nanasato et al., Tokyo Medical and Dental University searched the human cDNA database to screen for genes encoding secretory proteins having evident signal sequences. These genes were transfected into cultured vascular endothelial cells and then the culture supernatant was added to cultured vascular smooth muscle cells, so as to identify salusins as genes that induce increased intracellular Ca²⁺ concentration (see Non-Patent Document 2). Salusin-α comprises 28 amino acids and salusin-β comprises 20 amino acids. They are generated by processing of their precursor, preprosalusin. Preprosalusin is composed of 242 amino acids and is biosynthesized when the TOR2A gene, which is a related gene of TOR1A (DYT1) that has been reported as a causative gene of early-onset torsion dystonia, is subjected to selective splicing so that frame shift takes place. With a signal sequence of 26 amino acids on the N-terminus, it is inferred that two salusins are biosynthesized from the C-terminal side of 216-amino-acid Prosalusin. Distribution of expression of both salusin-α and salusin-β is confirmed in human vascular wall cells, endocrine-central nervous systems, and the like. Currently, it is possible to measure the concentration of salusin-α in human serum and the same in urine by radioimmunoassay. The results were 23.3 ± 8.1 pM and 156.8 ± 95.8 pM, respectively, in the case of healthy subjects (see Non-Patent Document 3). Major effects thereof have been confirmed in rats, including strong blood-pressure-lowering effects, strong heart rate-slowing effects β > α), vasopressin secretion-accelerating effects (only β) from the pituitary gland, and effects (β > α) of accelerating the proliferation of vascular smooth muscle cells and fibroblasts.
Non patent document 1: Shichiri M et al., Nat Med. 2003; 9: 1166-1172.
Non patent document 2: Masayoshi Nanasato, Journal of Experimental Medicine Vol. 210, No. 4 2004. 7. 24, pp. 267-270
Non patent document 3: Sato K et al., Peptides. 2006; 27: 2561-2566.

### Disclosure of the Invention

An object of the present invention is to provide a prophylactic and therapeutic agent for arteriosclerotic diseases. Another object of the present invention is to provide a method and a reagent for detecting arteriosclerotic diseases.

The present inventors have inferred that novel vasoactive materials, salusins, control of blood pressure, cardiac rate, and the like act on macrophages in a manner similar to that of known vasoactive materials, urotensin II and serotonin so as to have effects on foaming of macrophages; that is, they play a role in arteriosclerotic lesion formation. Hence, the present inventors have focused on the control of foaming of macrophages and expression regulation of ACAT-1, which is an essential enzyme for such control. The present inventors have also examined the effects of salusin-α and salusin-β. Thus, the present inventors have discovered that salusins play a pathophysiological role in arteriosclerotic lesion formation. Specifically, the present inventors have discovered that salusin-α suppresses foaming of macrophages, so as to suppress arteriosclerotic lesion formation, and that salusin-β accelerates foaming of macrophages, so as to accelerate arteriosclerotic lesion formation. The present inventors thus have further discovered that a compound capable of suppressing the effects of salusins-β or salusin-α can be used for preventing or treating arteriosclerotic diseases. Moreover, the present inventors have discovered that arteriosclerotic lesions are inversely correlated with the concentration of salusin-α in a biological sample and that salusin-α can be used as a marker for detection of arteriosclerotic diseases. Thus, the present inventors have completed the present invention.

The present invention is as follows.
[1] A therapeutic agent for an arteriosclerotic disease, comprising salusin-α or an agonist of salusin-α as an active ingredient.
[2] A therapeutic agent for an arteriosclerotic disease, comprising an antagonist of salusins-β as an active ingredient.
[3] The therapeutic agent for an arteriosclerotic disease according [2], wherein the antagonist of salusin-β is an anti-salusin-β antibody.
[4] The therapeutic agent for an arteriosclerotic disease according to any one of [1] to [3], wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.
[5] A therapeutic composition for an arteriosclerotic disease, comprising salusin-α or an agonist of salusin-α and an antagonist of salusin-β.
[6] The therapeutic composition for an arteriosclerotic disease according to [5], wherein the antagonist of salusin-β is an anti-salusin-β antibody.
[7] The therapeutic composition for an arteriosclerotic disease according to [5] or [6], wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.
[8] A method for detecting an arteriosclerotic disease, comprising assaying salusin-α in a biological sample.
[9] The method for detecting an arteriosclerotic disease according to [8], wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.
[10] The method for detecting an arteriosclerotic disease according to [8] or [9], wherein the biological sample is selected from the group consisting of serum, blood plasma, and urine.
[11] A method for determining the severity of an arteriosclerotic disease, comprising assaying salusin-α in a biological sample.
[12] The method for determining the severity of an arteriosclerotic disease according to [11], wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.
[13] The method for determining the severity of an arteriosclerotic disease according to [11] or [12], wherein the biological sample is selected from the group consisting of serum, blood plasma, and urine.
[14] A diagnostic marker for detecting an arteriosclerotic disease, comprising salusin-α.
[15] The diagnostic marker for detecting an arteriosclerotic disease according to [14], wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.
[16] A reagent for detecting an arteriosclerotic disease, comprising an anti-salusin-α antibody and using salusin-α as a diagnostic marker for detecting an arteriosclerotic disease.
[17] The reagent for detecting an arteriosclerotic disease according to [16], wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.

This description includes part or all of the contents as disclosed in the description and/or drawings of Japanese Patent Application No. 2007-127131, which is a priority document of the present application.

### Brief Description of the Drawings

Fig. 1 shows the relationship between salusin-α and salusins-β and their amino acid sequences.
Fig. 2A shows the dose-dependent effects of salusin-α and salusins-β on the acyl-CoA:cholesterol acyltransferase-1 (ACAT-1) protein expression in human monocyte-derived macrophages. Specifically, Fig. 2A shows the dose-dependent effects of salusin (0 nM to 0.6 nM).
Fig. 2B shows the dose-dependent effects of salusin α and salusin β on ACAT-1 protein expression in human monocyte-derived macrophages. Specifically, Fig. 2B shows the dose-dependent effects of salusin (0 nM to 10 nM).
Fig. 3 shows the time-dependent effects of salusin-α and salusin-β on ACAT-1 protein expression during the differentiation stage of human monocyte-derived macrophages.
Fig. 4 shows the principle for ACAT activity assay.
Fig. 5 shows the effects of salusin-α and salusin-β on the ACAT activity of human monocyte-derived macrophages.
Fig. 6 shows the effects of salusin-α and salusin-β on the ACAT-1 mRNA expression of human monocyte-derived macrophages.
Fig. 7 illustrates the principles behind cholesterol esterification assay. In Fig. 7, CE indicates cholesterol ester.
Fig. 8 shows the effects of salusin-α and salusin-β on the foaming of human monocyte-derived macrophages (accumulation of cholesterol ester [CE]).
Fig. 9 shows the principle for measurement of scavenger receptor class A (SR-A) activity.
Fig. 10 shows the effects of salusin-α and salusin-β on the scavenger receptor class A (SR-A) activity of human monocyte-derived macrophages. Fig. 10A shows the results of association assay and Fig. 10B shows the results of degradation assay.
Fig. 11 shows the effects of salusin-α and salusin-β on ATP-binding cassette transporter A1 (ABCA-1) protein expression in human monocyte-derived macrophages. Fig. 11A shows the results for salusin-α and Fig. 11B shows the results for salusin-β.
Fig. 12 shows the effects of salusin-α and salusin-β on ACAT-1 protein expression of differentiated cultured human monocyte-derived macrophages. Fig. 12A shows the results for salusin-α and Fig. 12B shows the results for salusins-β.

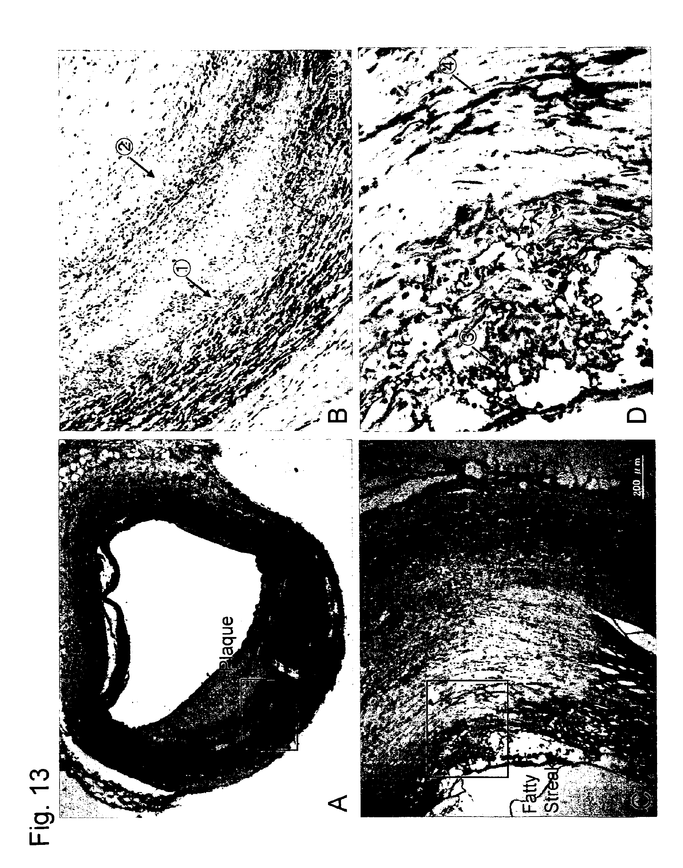

Fig. 13 shows photographs showing the expression of salusin-β in arteriosclerotic lesions of a human coronary artery. Photograph B is a highly-magnified image of A and photograph D is high-power image of C. Bars in Figs. 13A to D indicate 1.0 mm, 100 µm, 200 µm, and 200 µm, respectively. In the left anterior descending artery of a patient (72-year-old male) who died of acute coronary syndromes (ACS), strong expression of salusin-β was confirmed in macrophage-derived foam cells (3 in Fig. 13D) within fatty streaks of the right coronary artery and fibroblasts (4 in Fig. 13D) within tunica media, in addition to smooth muscle cells (1 in Fig. 13B) and fibroblasts (2 in Fig. 13B) in an atheroma (plaque) portion.
Fig. 14 shows the relationship between serum salusin-α concentrations in middle-aged male subjects and maximum value (maximum IMT) of intima-media complex thickness, which is an index of carotid sclerosis.
Fig. 15 shows comparison of serum salusin-α concentrations of patients with ischemic heart diseases (stable exertional angina pectoris, acute coronary syndromes [ACS], and old myocardial infarction), hypertension patients, and healthy subjects. The selected cases were stable exertional angina pectoris cases in which there had been no history of ACS, ACS cases in which examination took place within 6 hours after the onset of ACS, and old myocardial infarction cases in which the onset of ACS took place 6 or more months before being selected.
Fig. 16 shows serum salusin-α concentrations in the 1-vessel disease (1-VD) group, the 2-vessel disease (2-VD) group, and the 3-vessel disease (3-VD) group of acute coronary syndrome (ACS) patients.
Fig. 17-1 shows photographs showing the effects of salusin-β on arteriosclerotic lesions in the aorta of apoE-deficient mice. A-E show arteriosclerotic lesions in the aortic arch stained with oil red O. F-J show oil red O staining of proximal aorta cross-sections.
Fig. 17-2 shows the effects of salusin-β on arteriosclerotic lesions in the aorta of apoE-deficient mice. Mean ± SEM shown herein was found using 6 mice per group by measuring the area of an arteriosclerotic lesion of the aortic arch stained with oil red O.
Fig. 17-3 shows mean ± SEM of blood data obtained via measurement of 11 apoE-deficient mice per group.
Fig. 18-1 shows the effects of salusin-α on arteriosclerotic lesions in the aorta of apoE-deficient mice. A-C show arteriosclerotic lesions of thoracic aorta stained with oil red O. D-F show oil red 0 staining of proximal aorta cross-sections.
Fig. 18-2 shows the effects of salusin-α on arteriosclerotic lesions in the aorta of apoE-deficient mice. Mean ± SE shown herein was found using 5 mice per group by measuring the area of an arteriosclerotic lesion in the thoracic aorta stained with oil red O.
Fig. 18-3 shows mean ± SEM of blood data obtained via measurement of 8 apoE-deficient mice per group.
Fig. 19A shows foaming of macrophages in apoE-deficient mice. Data are each mean ± SEM obtained via measurement of 6 mice per group.
Fig. 19B shows the effects of salusin-α and salusin-β on cholesterol efflux in macrophages of apoE-deficient mice. Data are each mean ± SEM obtained via measurement of 6 mice per group.
Fig. 20A shows the effects of salusin-α and salusin-β on CD36 expression by macrophages of apoE-deficient mice. Data are each mean ± SEM obtained via measurement of 6 mice per group.
Fig. 20B shows the effects of salusin-α and salusin-β on SR-A expression by macrophages of apoE-deficient mice. Data are each mean ± SEM obtained via measurement of 6 mice per group.
Fig. 20C shows the effects of salusin-α and salusin-β on ACAT1 expression by macrophages of apoE-deficient mice. Data are each mean ± SEM obtained via measurement of 6 mice per group.
Fig. 20D shows the effects of salusin-α and salusin-β on ABCA1 expression by macrophages of apoE-deficient mice. Data are each mean ± SEM obtained via measurement of 6 mice per group.
Fig. 20E shows the effects of salusin-α and salusin-β on ABCG1 expression by macrophages of apoE-deficient mice. Data are each mean ± SEM obtained via assay of 6 mice per group.
Fig. 20F shows the effects of salusin-α and salusin-β on SR-BI expression by macrophages of apoE-deficient mice. Data are each mean ± SEM obtained via assay of 6 mice per group.

### Best Mode for Carrying Out the Invention

The present invention will be explained in detail as follows.

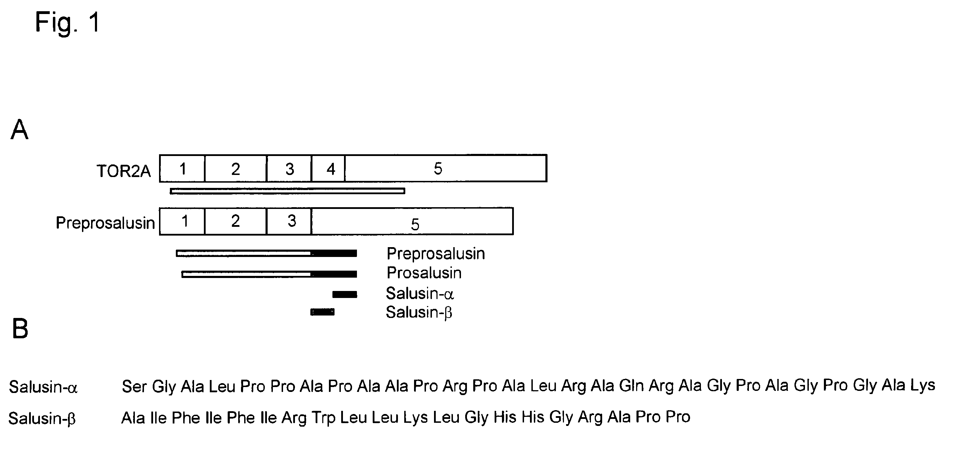

Salusin-α is a peptide (SEQ ID NO: 1) comprising 28 amino acid residues or a peptide (SEQ ID NO: 2) comprising 29 amino acid residues that have amidated glycine on the C-terminal side of the peptide comprising 28 amino acid residues. Also, salusin-β is a peptide (SEQ ID NO: 3) comprising 20 amino acid residues. Salusin-α and salusin-β are generated by processing from preprosalusin (SEQ ID NO: 4) comprising 242 amino acids. A signal sequence comprising 26 amino acids on the N-terminus of the preprosalusin is deleted to generate prosalusin comprising 216 amino acids, and then salusin-α and salusin-β are generated from the C-terminal side of prosalusin. Fig. 1 shows the relationship between salusin-α and salusin-β (Fig. 1A) and their amino acid sequences (Fig. 1B).

Salusin α suppresses foaming of macrophages, but salusin-β accelerates foaming of macrophages. Foaming of macrophages is a phenomenon that takes place due to incorporation of oxidized LDLs by macrophages, causing lipid deposition in vascular endothelium. Foaming of macrophages is caused by intracellular generation and accumulation of cholesterol ester. Oxidized LDLs are incorporated via various scavenger receptors of macrophages and then cholesterol ester produced from oxidized LDL-derived cholesterol is accumulated, so that foaming of macrophages takes place. After foaming of macrophages results in foam cells, primary lesions of arteriosclerotic diseases are formed, such as atheroma formation.

Salusin-α suppresses foaming of macrophages, so as to suppress formation of lesions of an arteriosclerotic disease. Hence, salusin-α can be used for preventing or treating arteriosclerotic diseases. Furthermore, an agonist of salusin-α can also be used for preventing or treating arteriosclerotic diseases in a manner similar to that of salusin-α. Here, examples of an agonist of salusin-α include all compounds that accelerate the effects of salusin-α. Examples of such agonist include a compound that binds to a receptor of salusin-α and thus exerts effects similar to those of salusin-α and an anti-salusin-α receptor antibody having agonistic activity.

On the other hand, salusin-β accelerates foaming of macrophages so as to act on arteriosclerotic lesion formation in an accelerated manner. Therefore, an antagonist of salusin-β that binds to salusin-β so as to suppress the effects of salusin-β suppresses the effects of salusin-β that cause foaming of macrophages, resulting in the ability to suppress formation of lesions of an arteriosclerotic disease. Hence, an antagonist of salusin-β can be used for preventing or treating arteriosclerotic diseases. Examples of such an antagonist of salusin-β in the present invention include all compounds such as neutralizing antibodies and receptor antagonists that bind to salusin-β or receptors of salusin-β so as to suppress the *in vivo* effects of salusin-β.

Diseases generalized as arteriosclerotic diseases are characterized by: increased Intima Media Thickness (IMT) due to risk factors of arteriosclerosis, which causes loss of elasticity and hardening of arteries; cholesterol ester deposition within arteries (atheroma formation) that narrows vascular passages (stenosis) or clogs the same (blockage); partial dilation of arterial walls (aneurysm); dilation of the entirety of arteries (arteriectasia); or cracks in the endothelia that causes dissociation of tunica media (dissociation) or rupture (bleeding), resulting in circulatory deficits throughout tissues and organs. In the present invention, prevention or treatment of arteriosclerotic diseases includes prevention or treatment of not only these arteriosclerotic diseases, but also acute coronary syndromes (ACS; acute coronary syndrome), an ischemic heart disease that is myocardial infarction or angina pectoris, brain infarction, encephalorrhagy, aortic aneurysm, aorta dissection, nephrosclerosis, arteriosclerosis obliterans, and the like caused by such arteriosclerotic diseases. Herein, the term "acute coronary syndromes" refers to pathological conditions resulting from thrombogenesis after the rupture of arteriosclerotic atheroma (plaque), followed by extremely decreased or blocked blood flow of coronary arteries. Clinical examples of such pathological conditions include unstable angina pectoris, acute myocardial infarction, and cardiac sudden death. Furthermore, arteriosclerosis is developed in relatively thick arteries such as the aorta, cerebral arteries, and coronary arteries. Examples of arteriosclerosis include atherosclerosis whereby atheromatous accumulation of cholesterol ester (melting point: 40°C) in arterial tunica intima causes atherosclerotic plaque formation and gradual increases in the thickness thereof, thus narrowing arterial lumen; arteriocapillary sclerosis that tends to occur in arteriolae in the brain or the kidneys due to changes caused by hypertension, which is associated with clogging (infarct) and bleeding due to the rupture of an entire vascular wall; and Monckeberg's arteriosclerosis caused by calcium deposition in arterial tunica media that hardens the artery and results in brittle tunica media. Moreover, the present invention can also be applied mainly to the prevention or treatment of the thickening of arterial walls or plaque formation.

Examples of an antagonist of salusin-β include all compounds that suppress the effects of salusin-β, such as an anti-salusin-β antibody having antagonistic activity.

Salusin-α can be prepared by chemical synthesis according to amino acid sequence information, or it can be prepared by genetic engineering techniques. Furthermore, a peptide consisting of an amino acid sequence that has a deletion, a substitution, or an addition of one or several amino acids with respect to the amino acid sequence of SEQ ID NO: 1 and having the activity of salusin-α can be used as the prophylactic or therapeutic agent for arteriosclerotic diseases of the present invention. Herein, the term "1 or several" refers to "1 to 3," preferably 1 or 2, and further preferably 1.

An anti-salusin-β antibody can be obtained by a known method as a polyclonal antibody or a monoclonal antibody and is preferably obtained as a monoclonal antibody. Examples of a monoclonal antibody include a monoclonal antibody produced by a hybridoma and a monoclonal antibody produced by a host transformed by a genetic engineering technique with an expression vector containing an antibody gene. A monoclonal antibody-producing hybridoma can be prepared by a known technique as described below. Specifically, a monoclonal antibody-producing hybridoma can be prepared by performing immunization by a known immunization method using salusin-β or a fragment peptide thereof as a sensitizing antigen, fusing the thus obtained immune cells to known parent cells by a general cell fusion method, and then screening for cells producing a monoclonal antibody by a known screening method. Upon immunization with salusin-β, a carrier protein such as bovine serum albumin (BSA) or keyhole limpet hemocyanin may be bound thereto and then used. A recombinant monoclonal antibody that can be used as a monoclonal antibody is produced by cloning an antibody gene from a hybridoma, incorporating the gene into an appropriate vector, introducing the vector into a host, and then causing the production of the antibody thereby via gene recombination techniques (for example, see Vandamme, A. M. et al., Eur. J. Biochem. 1990; 192: 767-775). At this time, DNA encoding an antibody heavy chain (H chain) and DNA encoding a light chain (L chain) may be separately incorporated into different expression vectors, and then host cells may be simultaneously transformed with the expression vectors. Alternatively, DNA encoding an H chain and an L chain is incorporated into a single expression vector, and then host cells may be transformed with the expression vector (see WO 94/11523). Furthermore, a recombinant antibody can also be produced using a transgenic animal. For example, an antibody gene is inserted into the middle of a gene encoding a protein uniquely produced in milk (e.g., goat β casein), so as prepare a fusion gene. A DNA fragment containing the fusion gene in which the antibody gene has been inserted is injected into a goat embryo and then the embryo is introduced into a female goat. A desired antibody can be obtained from milk that is produced by transgenic goats born from goats that have accepted such embryos, or from the progenies of such transgenic goats (Ebert, K. M. et al., Bio/Technology 1994; 12: 699-702).

Examples of the anti-salusin-β antibody of the present invention include gene recombinant antibodies artificially altered so as to lower heterologous antigenicity against humans, such as chimeric antibodies and humanized antibodies. Examples of such antibody include chimeric antibodies, humanized antibodies, and human antibodies, which can be all produced using known methods. A chimeric antibody can be obtained by preparing DNA encoding an antibody V region, ligating the DNA to DNA encoding a human antibody C region, incorporating the resultant into an expression vector, introducing the vector into a host, and then causing production thereof. A humanized antibody is also referred to as a human reshaped antibody. A humanized antibody is prepared by transplanting the complementarity determining region (CDR) of an antibody of a non-human mammal such as a mouse into the complementarity determining region of a human antibody. Such humanized antibody can be prepared by a known method (see European Patent Application Publication No. EP 125023 and WO 96/02576). Portions of a human antibody are used for the C region of a chimeric antibody or a humanized antibody. For example, Cγ1, Cγ2, Cγ3, and Cγ4 can be used for the H chain and Cκ and Cλ can be used for the L chain. Furthermore, to improve an antibody or the production stability thereof, the C region of a human antibody may be modified.

A human antibody can be obtained by introducing a human antibody gene locus, for example, and then administering an antigen to a transgenic animal capable of producing a human-derived antibody. Examples of the transgenic animal include mice. A method for producing mice capable of producing a human antibody is described in International Patent Publication WO02/43478 Pamphlet, for example.

Examples of the anti-salusin-β antibody of the present invention include not only complete antibodies, but also functional fragments thereof. The term "functional fragment of an antibody" refers to a portion (partial fragment) of an antibody that retains one or more effects of the antibody on an antigen. Specific examples thereof include F(ab')₂, Fab', Fab, Fv, disulfide bond Fv, single chain Fv (scFv), and polymers thereof [D. J. King., Applications and Engineering of Monoclonal Antibodies, 1998 T. J. International Ltd].

Whether or not the thus obtained antibody has antagonist activity against salusin-β can be determined by binding an antibody to salusin-β and then verifying whether or not the activity of salusin-β is suppressed, for example.

A compound having antagonistic activity against salusin-α or salusin-β can be used as a prophylactic or a therapeutic agent for arteriosclerotic diseases.

The dose varies depending on symptoms, age, sex, and the like. Generally, in the case of oral administration, a dose ranging from approximately 0.01 mg to 1000 mg per day for an adult and can be administered once or several separate times. In the case of parenteral administration, a dose ranging from approximately 0.01 mg to 1000 mg per administration can be administered via subcutaneous injection, intramuscular injection, or intravenous injection. Moreover, the timing for administration may be either before or after the occurrence of clinical symptoms of arteriosclerotic diseases.

The prophylactic or therapeutic agent of the present invention can be administered in various forms. Examples of the routes for administration of such forms include oral administration of tablets, capsules, granules, powder, syrups, and the like and parenteral administration of injection preparations, drops, suppositories, and the like.

The prophylactic or therapeutic agent for arteriosclerotic diseases containing a compound having antagonistic activity against salusin-α or salusin-β of the present invention can be formulated according to a standard method (Remington's Pharmaceutical Science, latest edition, Mark Publishing Company, Easton, U.S.A.), and such compound may contain a pharmaceutically acceptable carrier and a pharmaceutically acceptable additive together. Examples of such carrier and pharmaceutical additive include water, a pharmaceutically acceptable organic solvent, collagen, polyvinyl alcohol, polyvinylpyrrolidone, carboxy vinyl polymer, sodium carboxymethylcellulose, sodium polyacrylate, sodium alginate, water-soluble dextran, sodium carboxymethyl starch, pectin, methylcellulose, ethyl cellulose, xanthan gum, gum Arabic, casein, agar, polyethylene glycol, diglycerin, glycerin, propylene glycol, vaseline, paraffin, stearyl alcohol, stearic acid, human serum albumin (HSA), mannitol, sorbitol, lactose, and a surfactant acceptable as a pharmaceutical additive. Actual additives are independently selected, or an appropriate combination thereof is selected from the above examples depending on the formulation of the prophylactic or therapeutic agent of the present invention. However, examples thereof are not limited thereto.

Further, the prophylactic or therapeutic agent for arteriosclerotic diseases of the present invention may contain a combination of a compound having salusin-β antagonistic activity in combination with salusin-α.

The present invention further encompasses the following methods involving the assay of salusin-α in a biological sample: a method for detecting whether or not a test subject from which a biological sample has been collected contracts an arteriosclerotic disease, a method for determining the severity of an arteriosclerotic disease, and a method for evaluating a risk of contracting an arteriosclerotic disease. In particular, the present invention can be preferably used for detection of acute coronary syndromes.

Examples of a biological sample include whole blood, serum, blood plasma, and urine. Salusin-α contained in such a biological sample can be quantitatively or qualitatively assayed.

Alternatively, a biological sample can be directly used for the assay of salusin-α therein. However, since the salusin-α content in a biological sample is low, it is preferable to concentrate or isolate salusin-α from a biological sample for assay. Such isolation or concentration can be carried out via solid phase extraction from serum, blood plasma, urine, or the like. Herein, the term "solid phase extraction" refers to a technique for exclusively extracting specific target components in a selective manner from a sample with a complicated composition with the use of a stationary phase (solid phase or adsorbent) such as chemically-bound silica gel, porous polymer, alumina, activated carbon, or the like for separation/purification, whereby contaminants in the sample can be removed. For solid-phase extraction, a stationary phase for reversed-phase partition chromatography, stationary phase for ion exchange chromatography, and the like can be widely used. For a stationary phase for reverse phase chromatography, filling agents having groups such as octadecyl silyl groups (C18), octyl groups (C8), butyl groups (C4), trimethyl groups (C3), phenyl groups (Ph), butyl groups, triacontyl groups (C30), and the like can be used. Of these, a filling agent having octadecyl silyl groups (C18) or octyl groups (C8) is preferable. A commercially available cartridge is used for solid phase extraction. A syringe type solid phase cartridge or a disc type solid phase cartridge can be used. Examples of a commercially available cartridge include Sep-Pak (registered trademark, Waters), Autoprep (registered trademark, Showa Denko K.K.), Discovery DSC-18 (SUPELCO), and Discovery DSC-8 (SUPELCO). Examples of a solvent used for elution of salusin-α include acetonitrile, methanol, and tetrahydrofuran. A biological sample is subjected to acid treatment with trifluoroacetic acid (TFA) prior to solid phase extraction, followed by centrifugation. The thus obtained supernatant may be used.

In addition, a pretreated biological sample may be subjected to isolation and purification by HPLC with the use of a reverse phase chromatography column.

Salusin can be assayed by immunoassay such as radioimmunoassay (RIA), enzyme immunoassay (EIA), ELISA, or fluorescence assay. Upon assay, an anti-salusin-α antibody labeled with a radioisotope such as ¹²⁵I, an enzyme such as alkaline phosphatase, horseradish peroxidase, or β-galactosidase, or a fluorescent substance such as Fluorescein Isothiocyanate (FITC) may be used. Such immunological assay can be carried out by a known method. For instance, a primary ligand capable of binding to salusin-α such as an anti-salusin-α antibody is immobilized on a solid-phase support in wells of a plastic-made microtiter plate well or the like. Then, a biological sample is allowed to react with the primary ligand capable of binding to salusin-α such as an anti-salusin-α antibody bound to the solid phase. Accordingly, salusin-α in the sample is allowed to bind to the solid phase via the primary ligand bound to the solid phase as a result of a ligand-receptor binding reaction such as an antigen-antibody reaction. Subsequently, washing is carried out, and then the second ligand capable of binding to salusin-α is allowed to react with salusin-α in the sample bound to the solid phase. Specifically, a complex of the primary ligand capable of binding to salusin-α, such as an anti-salusin-α antibody, which has been bound to a solid phase / salusin-α / the second ligand is formed. An anti-salusin-α antibody or the like can be used as a second ligand capable of binding to salusin-α. Subsequently, washing is carried out and then the second ligand capable of binding to salusin-α, such as a second anti-salusin-α antibody, which has been bound to the solid phase, is assayed. Such assay can be carried out by a variety of methods that can be generally used in the field of immunological analysis. For instance, the second ligand capable of binding to salusin-α is labeled with an enzyme, fluorescence, biotin, a radioactive label, or the like so as to produce, for example, an enzyme-labeled product. A second ligand capable of binding to salusin-α, which has been bound to a solid phase, can be assayed by assaying such label.

Upon assay of salusin-α, not only intact salusin-α but also a salusin-α fragment can be assayed.

An example of a technique for isolating and purifying salusin-α from a biological sample is described below.
(1) Trifluoroacetic acid (0.1% (v/v)) is added to serum or blood plasma (2 mL), followed by centrifugation at 3000 rpm for 10 minutes.
(2) The obtained supernatant is introduced into a Sep-Pak (registered trademark) C18 cartridge. The cartridge is preliminarily treated with 99.8% methanol (5 mL), pure water (5 mL), and 0.1 % TFA (5 mL).
(3) Extraction is carried out with 0.1% TFA (1mL) and 50% methanol. The obtained eluate is concentrated to approximately 100 µL with the use of a centrifugal concentrator at 2000 rpm.
(4) An assay buffer (250 µL) is added to a sample to assay salusin-α contained therein.

A method for isolation and purification for assay of salusin-α and an RIA assay method can be carried out according to Sato K et al., Peptides, 2006; 27: 2561-2566.

The salusin-α concentrations in human serum and urine were 23.3 ± 8.1 pM and 156.8 ± 95.8 pM, respectively, for a healthy subject (mean ± SD for each value). Therefore, when the salusin concentration in serum or urine for a test subject is significantly lower than that for a healthy subject, it can be judged that the test subject is contracting an arteriosclerotic disease or is at a significant risk of contracting an arteriosclerotic disease. An example of a case in which the salusin concentration is significantly low is a case in which the salusin concentration is less than mean - 1 × SD, preferably less than mean - 2 × SD, and further preferably less than mean - 3 × SD when compared with the aforementioned mean and SD for a healthy subject. In addition, the greater the severity of an arteriosclerotic disease, the lower the salusin-α concentration. For instance, in the case of acute coronary syndromes (ACS), the salusin-α concentration in a biological sample decreases in a test subject as the degree of lesion progress increases in the order of the 1-vessel coronary artery disease group, the 2-vessel coronary artery disease group, and the 3-vessel coronary artery disease group. In addition, there is a negative correlation between the salusin-α concentration in a biological sample and the thickness of an intima-media complex (IMT: Intima Media Thickness) serving as an arteriosclerosis index. The thicker the IMT, the lower the salusin-α concentration. Therefore, salusin-α can replace IMT so as to serve an index of progress in an arteriosclerotic disease. Further, salusin-α is effective for detection of an acute arteriosclerotic disease.

Furthermore, management of the progress of an arteriosclerotic disease can be carried out through regular assay of salusin-α in a biological sample collected from a test subject and monitoring of the salusin-α concentration.

Salusin-α can be used as a marker for detection/diagnosis of an arteriosclerotic disease. The present invention encompasses the use of salusin-α as a detection marker for detecting an arteriosclerotic disease and further encompasses a disease detection/diagnosis marker comprising salusin-α for detection/diagnosis of an arteriosclerotic disease.

The present invention is hereafter described in greater detail with reference to the following examples, although the technical scope of the present invention is not limited thereto.

In the Examples below, monocytes were prepared from human peripheral blood and cultured for 7 days so as to have differentiated into macrophages. At such time, salusin-α (0 to 10 nM) and salusin-β (0 to 10 nM) were simultaneously added thereto, and the effects of salusin were examined.

Culture of monocytes obtained from human peripheral blood was performed by a known method. Isolated monocytes were cultured with the addition of RPMI-1640 (15 mL) and then the floating monocytes were subjected to cell counting with a blood cell counting chamber. The cells were seeded on a 6-cm dish so as to result in 4 × 10⁶ cells/2 mL. The monocytes were kept warm at 37°C in the presence of 5% CO₂ for 1 hour such that the cells were allowed to adhere to the dish. The medium was substituted with RPMI-1640 containing 10% human serum, followed by culture for 7 days. The medium was changed every 3 days.

### Example 1: Effects of salusin-α and salusin-β on ACAT-1 protein expression in human monocyte-derived macrophages

ACAT-1 is an endoplasmic reticulum enzyme that converts intracellular free cholesterol into cholesterol ester. The ACAT-1 gene is important for foaming of macrophages. In order to examine effects of salusin-α and salusin-β on the ACAT-1 expression, the ACAT-1 protein expression was examined by Western blotting.

Monocyte-derived macrophages that had been cultured for 7 days were washed with PBS 3 times, followed by solubilization with the addition of 10% SDS (sodium dodecyl sulfate) (100 µL) to each dish. Each cell extraction solution was scooped up with a cell scraper, followed by pulverization with the use of a 1-mL TERUMO syringe and a 23-G injection needle for fragmentation (shearing). The resultant was centrifuged at 15,000 rpm for 3 minutes (Himac CF15D2 high-speed micro refrigerated centrifuge; Hitach, Ltd.). The supernatant protein concentration was assayed by the bicinchoninic acid (BCA) method (Pierce) and used as a cell extraction solution for Western blotting.

ACAT-1 or ABCA-1 in each culture supernatant obtained in the Examples of the present invention was assayed by Western blotting according to the method described below.

### ACAT-1 protein detection

For ACAT-1 protein detection, an anti-human ACAT-1 polyclonal antibody (DM10 donated from Dr. Chang, Dartmouth Medical School) [Chang CCY et al., J Biol Chem. 1995; 270: 29532-29540] prepared by immunizing a rabbit was used as a primary antibody (final concentration: 0.5 µg/mL) for a reaction on a transfer membrane at room temperature for 1 hour. The transfer membrane was washed with PBS-0.1 % Tween 20, followed by a reaction with a secondary antibody (anti-rabbit IgG antibody: anti-rabbit IgG donkey antibody conjugated with horseradish peroxidase (HRP) (GE Healthcare Bio-Science)) at room temperature for 1 hour. For a secondary antibody, a stock solution (0.5 µL) was diluted 10,000-fold with PBS-0.1% Tween 20 (5 mL), and the resultant was used. Washing was carried out with PBS-0.1% Tween 20, followed by ACAT-1 protein detection with the use of Lumi-Light Plus (PerkinElmer Life Sciences).

### ABCA-1 protein detection

For ABCA-1 protein detection, a reaction with an anti-human ABCA-1 antibody was carried out on a transfer membrane at room temperature for 1 hour. For a primary antibody (anti-human ABCA-1 rabbit polyclonal antibody (Funakoshi Corporation)), a stock solution (5 µL) was diluted 1,000-fold with PBS-0.1% Tween 20 (5 mL), and the diluent was used. The transfer membrane was washed with PBS-0.1% Tween 20. Then, a reaction with a secondary antibody (anti-rabbit IgG antibody: anti-rabbit IgG donkey antibody conjugated with horseradish peroxidase (HRP) (GE Healthcare Bio-Science)) was carried out at room temperature for 1 hour. For a secondary antibody, a stock solution (0.5 µL) was diluted 10,000-fold with PBS-0.1% Tween 20 (5 mL), and the diluent was used. Washing was carried out with PBS-0.1% Tween20, followed by ABCA-1 protein detection with the use of Lumi-Light Plus (PerkinElmer Life Sciences).

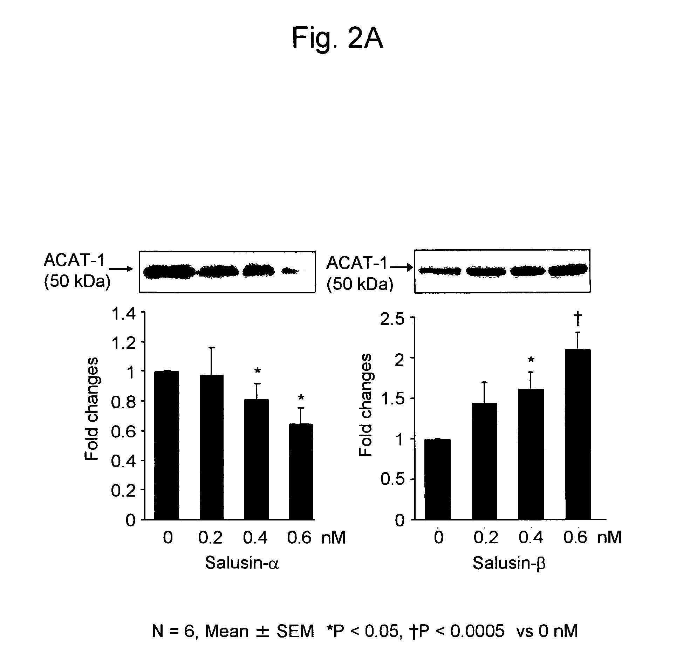

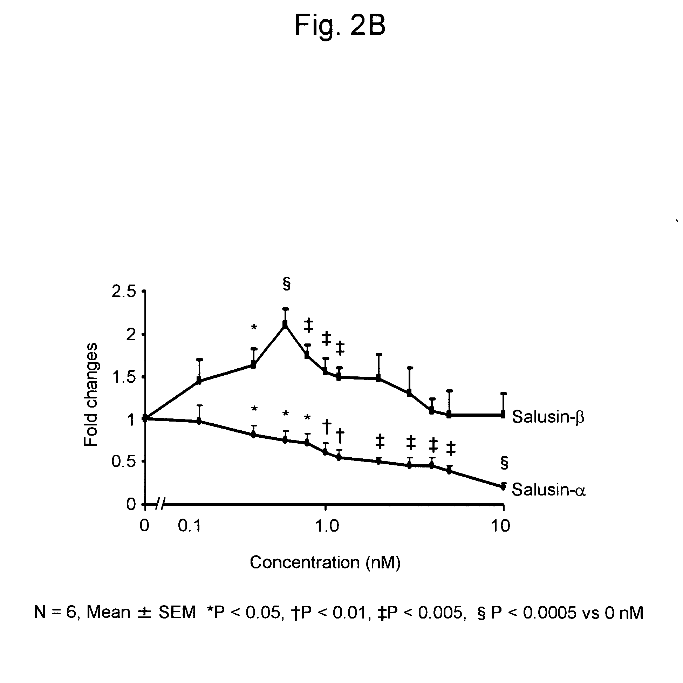

Human peripheral blood monocytes (4 × 10⁶ cells/6-cm dish) were cultured for 7 days in an RPMI-1640 medium (2 mL) containing 10% human serum so as to differentiate into macrophages. In order to examine the salusin dose-dependent effects on the ACAT-1 protein expression, salusin-α or salusin-β was added to the medium at a concentration of 0, 0.2, 0.4, 0.6, 0.8, or 1.0 nM at the beginning of culture. On Day 7 of culture, macrophage cells differentiated from monocytes were washed with PBS and then solubilized in 10% SDS (100 µL), followed by Western blotting with the use of an ACAT-1-specific antibody. As a result, salusin-α was found to have inhibited approximately 40% of ACAT-1 protein expression in a concentration-dependent manner (Fig. 2A). Meanwhile, salusins-β promoted ACAT-1 protein expression in a concentration-dependent manner at concentrations from 0 to 0.6 nM. Effects of salusin-β reached the peak level at 0.6 nM, at which the ACAT-1 protein expression level increased to 2.1 times as great as that of a control. The ACAT-1 protein expression enhancement effects of salusins-β were gradually attenuated at concentrations of more than 0.6 nM (Fig. 2B).

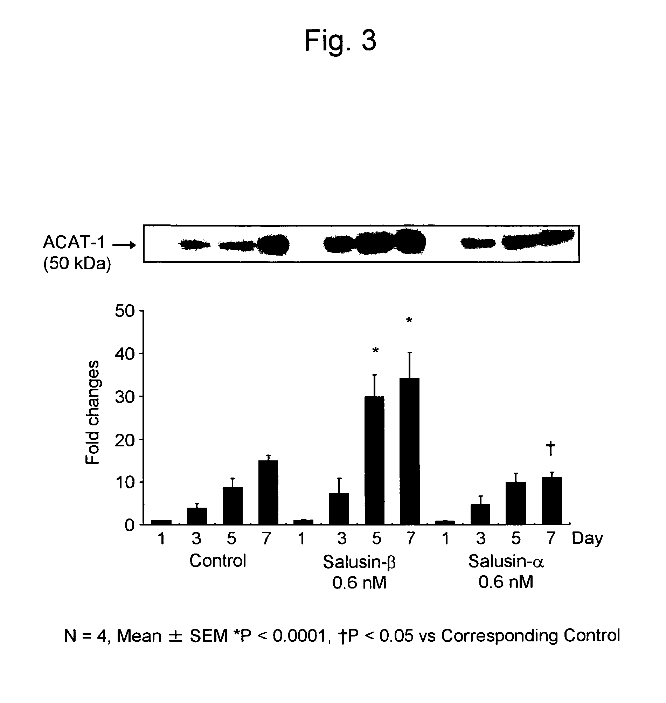

Next, time-dependent effects of salusin-α or salusin-β on ACAT-1 protein expression during the course of differentiation from monocytes into macrophage differentiation were examined. Human peripheral blood monocytes (4 × 10⁶ cells/6 cm dish) were cultured for 7 days so as to differentiate into macrophages. At such time, 0.6 nM salusin-α or salusin-β was added during culture. Each dish was washed with PBS on Days 1, 3, 5, or 7 after seeding. The dishes in which reaction had been discontinued on Day 1, 3, or 5 were temporarily frozen at -80°C and thawed on Day 7. The thus obtained cells and the cells on Day 7 after seeding were solubilized in 10% SDS (100 µL). As a result of examination by the Western blotting method, ACAT-1 protein expression in the cells treated with salusin-α on Day 1, 3, or 5 after seeding was not found to significantly differ from that in the control cells. However, the expression was significantly inhibited on Day 7. The level of ACAT-1 protein expression in the cells treated with salusin-β was found to have significantly increased to more than the level of the same in control cells on Day 5 or later (Fig. 3).

### Example 2: Effects of salusin-α and salusin-β on ACAT activity in human monocyte-derived macrophages

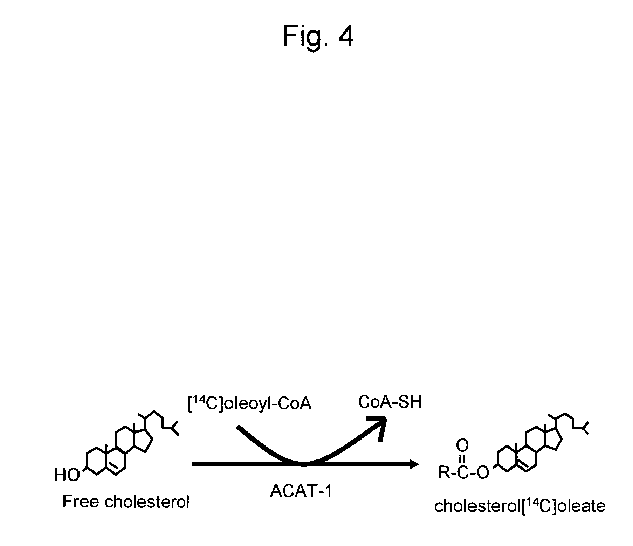

For ACAT activity assay, the liposome reconstitution method was used. This method involves the use of a system in which the ACAT-1 protein present in the endoplasmic reticulum is reconstituted into liposomes with the use of mixed micelles comprising cholesterol/phosphatidylcholine, thereby achieving a saturated concentration level of cholesterol that can be used as an ACAT-1 substrate [Cheng D et al., J Biol Chem. 1995; 270: 685-695.]. In the case of the conventionally used microsome method, cholesterol that endogenously exists in the endoplasmic reticulum has been used as a substrate. However, in general, the endoplasmic reticulum membrane is poor in cholesterol. Therefore, it is thought that the saturated level of cholesterol capable of serving as an ACAT substrate has not been achieved, resulting in underestimation of ACAT activity. Herein, ACAT activity was assayed by adding another substrate, [¹⁴C]oleoyl-CoA, to a reaction system and assaying the cholesterol [¹⁴C]oleate generated through the ACAT reaction (Fig. 4).

The cell extraction solution used in this Example was prepared as described below.

Peripheral blood human monocytes (4 × 10⁶ cells/2 mL) were seeded on 4 to 6 6-cm dishes and cultured for 7 days so as to differentiate into macrophages. At such time, salusin-α or salusin-β was added during culture. Then, effects of salusin-α or salusin-β on ACAT activity were examined. After culture for 7 days, the cells were washed twice with PBS that had been cooled to 4°C, a hypotonic buffer (1 mM Tris-HCl, 1 mM EDTA, pH 7.4) was added thereto (2 mL/dish), and the resultant was left for 5 minutes. These operations were intended to pulverize cell membranes by osmotic pressure, wash the discharged cytoplasm protein, and concentrate the membrane protein containing ACAT-1. The dishes were turned upside down in order to sufficiently remove the buffer therefrom. Buffer A (50 mM Tris-HCl, 1 mM EDTA, pH 7.4) (100 µL) containing a protease inhibitor cocktail (SIGMA) (1/1000 volume) was added to one of the dishes immediately before use. The cells were recovered therefrom with a cell scraper. The buffer (100 µL) was transferred to the next dish subjected to culture under the same conditions, followed by the same operations. At the end, the 4 to 6 dishes were united so as to concentrate the membrane protein. The recovered cell extraction solution (1 mL) was introduced into a Teflon homogenizer, followed by 3 sets of homogenization (20 strokes per set). Homogenization was performed on ice with the break time between sets being approximately 1 minute. The protein concentration of the homogenized cell extraction solution was quantitatively assayed by the BCA method (Pierce). The cell extraction solution was mixed with 4 M KCl (1/3 volume) and 20% CHAPS (1/9 volume) (such that the final concentration of KCl was 1M and the same of CHAPS was 2%). The protein concentration was adjusted with buffer A containing 1 M KCl and 2 % CHAPS. The resultant was left at room temperature for 10 minutes.

Mixed micelles were prepared as described below.

100 mg/mL L-α-phosphatidylcholine from egg yolk (SIGMA) in chloroform (172.4 µL) and 20 mg/mL cholesterol (SIGMA) in benzene (62 µL) were introduced into a scintillation vial and vortexed, followed by drying with nitrogen gas. 10% taurocholic acid (SIGMA) (100 µL), 10 × buffer A (100 µL), and sterilized purified water (800 µL) were added thereto, and the resultant was vortexed. The air in the vial was substituted with nitrogen gas, followed by sonication for 10 minutes and cooling on ice for 5 minutes while shielding light. The above operations were repeated approximately 10 times so as to obtain a substantially transparent resultant. After sonication, sterilized purified water (1 mL) was added thereto, followed by sufficient mixing.

A 0.25 mM [¹⁴C]oleoyl-CoA mixture was obtained by preparing a 2,000-µL solution containing 1 mM [¹⁴C]oleoyl CoA (GE Healthcare Bio-Science, CFA634, 1.85 MBq) (16 µL), 34 mM cold oleoyl-CoA (SIGMA) (10 µL), 30 mg/mL BSA in 0.25 M Tris-HCl (pH 7.8) (834 µL), and sterilized purified water (996 µL).

Thin layer chromatography (TLC) was performed as described below.

A TLC standard was prepared by adding cholesterol oleate (SIGMA) (20 mg) to isopropanol (4 mL), heating the mixture in a hot bath at 60°C for solubilization, and further adding 0.91 g/mL glyceryl trioleate (SIGMA) (20 µL) thereto. The TLC standard (60 µL) was spotted by capillary spotting on silica gel 60 F₂₅₄ (Merck) on TLC aluminum sheets (20 × 20 cm). Hexane/diethylether/acetic acid (90:10:1, v/v) was prepared as a development solvent.

Mixed micelles (140 µL) were added to a cell extraction solution (80 to 100 µg) with a constant protein amount, and the resultant was slowly mixed and allowed to stand on ice for 10 minutes. Then, a [¹⁴C]oleoyl-CoA mixture (20 µL) was added thereto, followed by mixing without causing foaming. The resultant was kept warm at 37°C for 30 minutes for reaction. Thereafter, chloroform/methanol (2:1, v/v) (3 mL) and sterilized purified water (1 mL) were added thereto, and the resultant was vortexed so as to terminate the reaction. Centrifugation was performed at 2,500 rpm for 10 minutes (TOMY LC-120 low-speed centrifuge, rotor 7015-06). Then, the top layer (water layer) was removed therefrom and the bottom layer (oil layer) was dried with nitrogen gas. The extracted lipid was dissolved with isopropanol (60 µL). The resultant was spotted between standard spots. The lipid was developed until the development solvent reached the top edge of the TLC plate in a glass chamber. The TLC plate was transferred to another glass chamber in which a small amount of iodine had been dispersed for color development of lipids of the TLC standard. The portion corresponding to cholesterol oleate was excised with scissors and placed in a vial, followed by assay with the addition of a scintillation cocktail (Perkin Elmer) (3 mL). For specific activity assay, the radioactive activity of the [¹⁴C]oleoyl-CoA mixture (10 µL) was assayed. The ACAT activity level (pmol/mg cell protein/min) was calculated by dividing the radioactive activity (cpm) of cholesterol [¹⁴C]oleate generated through the ACAT reaction by the amount of cell protein used in the reaction system (mg), the specific activity (cpm/pmol) of [¹⁴C]oleoyl-CoA, and the reaction time (10 minutes).

Salusin-α or salusin-β were found to have caused changes in ACAT-1 protein expression. Therefore, effects of salusin-α or salusin-β on the ACAT enzyme activity were examined. Human peripheral blood monocytes (4×10⁶ cells/dish) were cultured in an RPMI-1640 medium (2 mL) containing 10% human serum for 7 days so as to differentiate into macrophages. At such time, 0.6 nM salusin-α or salusin-β was added to the medium during culture.

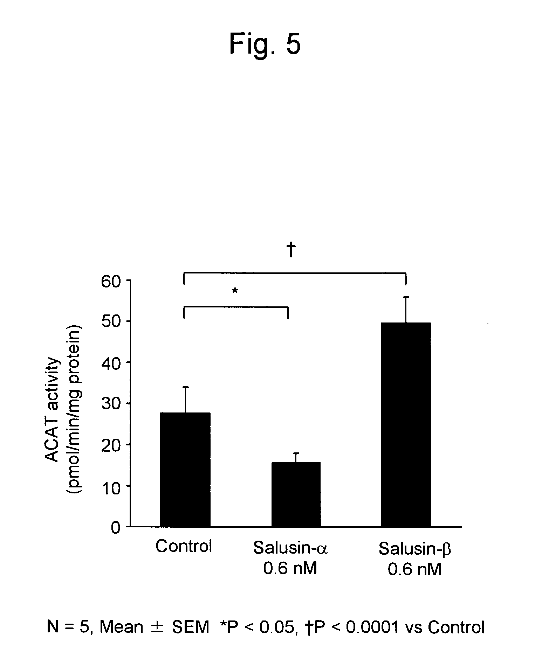

The ACAT-1 protein was reconstituted into liposomes by adding mixed micelles (cholesterol/phosphatidylcholin) to the cell extraction solution. [¹⁴C]oleoyl-CoA was added to the resulting sample, followed by reaction at 37°C for 10 minutes. The radioactive activity of cholesterol [¹⁴C]oleate generated through the ACAT reaction was assayed and then the ACAT activity was calculated. As a result, the ACAT activity in the cells treated with salusin-α was found to have been suppressed to 50% of that of the control. Meanwhile, the ACAT activity in the cells treated with salusin-β was found to have increased to a level 1.8 times as great as that of the control (Fig. 5).

### Example 3: Effects of salusin-α and salusin-β on ACAT-1 mRNA expression in human monocyte-derived macrophages

RNA extraction was performed using a TRIzol reagent (LIFE TECHNOLOGIES).

Cells were cultured for 7 days in a medium and then the medium was removed. The cells were washed 3 times with PBS. PBS was sufficiently removed therefrom and TRIzol (1 mL) was added thereto for cell lysis. The cell extraction solution was pippeted in and out 5 times and then transferred to a new Eppendorf tube. Then, the tube was left at room temperature for 5 minutes. Chloroform (200 µL) was added thereto, followed by strong shaking for 15 seconds. The tube was further left for 2 to 3 minutes and centrifuged at 12,000 rpm for 15 minutes (Himac CF15D2 high-speed micro refrigerated centrifuge; Hitach, Ltd.). The top layer was transferred to a new Eppendorf tube and isopropanol (500 µL) was added thereto, followed by end-over-end mixing. The tube was left for 10 minutes and centrifuged at 12,000 rpm for 10 minutes. The supernatant was removed from the resultant. 75% ethanol (180 µL) was added to the remaining precipitation, followed by centrifugation at 15,000 rpm for 5 minutes. The supernatant was removed from the resultant, followed by air drying. The resultant was dissolved in sterilized purified water (90 µL) and kept warm at 65°C for 10 minutes. A DNase I solution (10 µL) was added thereto, and the resultant was kept warm at 37°C for 15 minutes. 3M sodium acetate (10 µL) and phenol/chloroform (1:1, v/v) (100 µL) were added thereto followed by shaking. Centrifugation was performed at 15,000 rpm for 3 minutes. The supernatant was collected in a new Eppendorf tube. Chloroform/isoamyl alcohol (24:1, v/v) (100 µL) was added thereto, and the tube was further centrifuged at 15,000 rpm for 3 minutes. The supernatant was collected and ethanol (200µL) was added thereto. The resultant was allowed to stand at -80°C for 30 minutes or more, followed by centrifugation at 15,000 rpm for 10 minutes. The supernatant was removed. The resultant was washed with 70% ethanol and dried. The obtained product was dissolved in sterilized distilled water (15 µL). The concentration of the thus obtained RNA solution was obtained by absorbance determination at 260 nm. Sterilized purified water was added to the solution to achieve the predetermined concentration.

Next, the RNA expression of ACAT-1 mRNA was analyzed by the real time RT-PCR method.

Primer sequences were determined Primer Express Versionl.0 (PE Biosystems) with the use of the respective human mRNA sequences shown below.
β-action
Forward Primer: 5'-CCTGGCACCCAGCACAAT (SEQ ID NO: 5)
Reverse Primer: 5'-GGGCCGGACTCGTCATAC (SEQ ID NO: 6)
ACAT-1
Forward Primer: 5'-TTCGGAATATCATCAAACAGGAGCC (SEQ ID NO: 7)
Reverse Primer: 5'-CACACCTGGCAAGATGGAGTT (SEQ ID NO: 8)

Peripheral blood human monocytes (4 × 10⁶ cells/6 cm dish) treated with salusin-α or salusin-β were cultured in an RPMI-1640 medium (2 mL) containing 10% human serum for 7 days so as to differentiate into macrophages. Total RNA was extracted from cells treated with salusin-α or salusin-β and from untreated cells. Each total RNA (1 µg) was subjected to a reverse transcription reaction for cDNA synthesis.

Real time PCR was performed to quantify the obtained cDNA by the GeneAmp 5700 Sequence Detection System (Applied Biosystems Japan) with the use of qPCR MasterMix SYBR GreenI (Eurogentec).

Next, a reverse transcription reaction was carried out by the method described below.

Random primers (TaKaRa, 80 nM) (0.5 µL) and sterilized purified water (10.5 µL) were added to each total RNA solution (1 µg/µL) (1 µL), followed by heating at 70°C for 10 minutes. An RNase inhibitor (0.75 µL), 5 × First Strand Buffer (Invitrogen) (4 µL), 0.1 M dithiothreitol (2 µL), 2.5 mM dNTP (TaKaRa) (4 µL), and SuperScript II (200 U/µL; Invitrogen) (1 µL) were added thereto, followed by a reaction at 37°C for 1 hour. After denaturation at 95°C for 5 minutes, the resultant was cooled on ice and sterilized purified water (40 µL) was added thereto.

Real time RT-PCR was carried out by the method described below.

qPCR MasterMix SYBR GreenI (12.5 µL), a primer solution (Forward + Reverse: 5 µM each) (2.5 µL), and sterilized purified water (8 µL) were added to the cDNA solution (2 µL) obtained by the reverse transcription reaction, followed by reaction.

PCR cycles included a cycle of 95°C for 10 minutes and 40 cycles of 94°C for 20 seconds, 55°C for 20 seconds, and 72°C for 30 seconds.

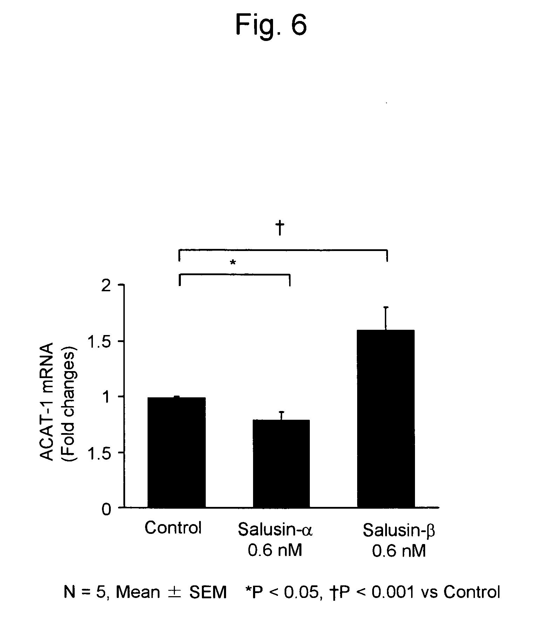

Salusin-α and salusin-β were found to have caused changes in the ACAT-1 protein expression and ACAT activity. Therefore, effects of salusin-α and salusin-β on ACAT-1 mRNA expression were further examined. Human peripheral blood monocytes (4 × 10⁶ cells/dish) were cultured in an RPMI-1640 medium (2 mL) containing 10% human serum for 7 days so as to differentiate into macrophages. At such time, 0.6 nM salusin-α or salusin-β was added during culture for examination of effects of salusin-α or salusin-β on ACAT-1 mRNA expression. Total RNA was extracted from cells on Day 7 of culture. Effects of salusin-α and salusin-β on ACAT-1 mRNA were examined by real-time RT-PCR. Accordingly, salusin-α was found to have suppressed the ACAT-1 mRNA expression level to 80% of that of the control, and salusin-β was found to have promoted the ACAT-1 mRNA expression level to 1.6 times as great as that of control (Fig. 6).

### Example 4: Effects of salusin-α and salusin-β on foaming of human monocyte-derived macrophages (cholesterol ester accumulation)

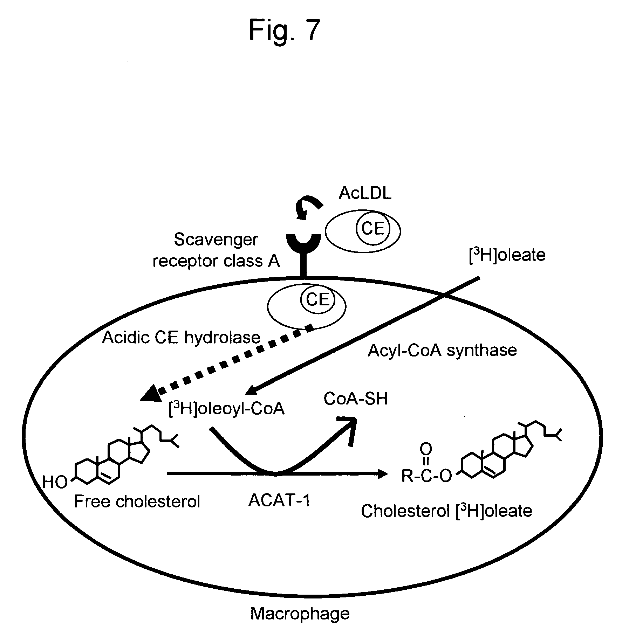

Cholesterol esterification assay was employed as a method for assaying foaming of macrophages [Goldstein JL et al., Methods Enzymol. 1983; 98: 241-260]. Macrophages cause acetylated LDL (AcLDL) to be incorporated into cells via scavenger receptors so as to result in lysosome degradation. Free cholesterol produced in a lysosome is transferred to ACAT and converted into cholesterol ester as a result of ACAT reaction. If [³H]oleate is added to a medium, [³H]oleate is incorporated into cells and converted into [³H]oleoyl-CoA by acyl CoA synthase. ACAT produces cholesterol [³H]oleate with the use of, as substrates, AcLDL-derived free cholesterol and [³H]oleoyl-CoA obtained as above. The radioactivity of cholesterol [³H]oleate accumulated in cells can serve as a sensitive indicator for cholesterol ester accumulation (Fig. 7).

Human blood plasma LDL was purified by the method described below.

LDL (d = 1.019-1.063) was purified from human blood plasma by sequential flotation ultracentrifugation [Schumaker VN et al., Methds Enzymol. 1986; 128: 155-170]. Human blood plasma (350 mL) was dispensed into 6 centrifuge tubes subjected to ultracentrifugation at 100,000 x g (36,000 rpm) at 4°C for 20 hours with the use of an RP-42 Rotor. The cloudy top layer containing chylomicron and very low-density lipoportein (VLDL) was removed from the resultant and the bottom layer (yellow layer) was recovered using an aspirator. The volume of the obtained blood plasma fraction from which chylomicron and VLDL had been removed was measured with a volumetric flask. The specific gravity thereof was assayed with a floating hydrometer. Solid KBr was added thereto until the condition of "d = 1.063" was achieved. Assuming "V" denotes the volume of the obtained blood plasma fraction from which chylomicron and VLDL had been removed and "D" denotes the specific gravity thereof, the amount of KBr that must be added in order to achieve a specific gravity of 1.063 g/mL can be approximated by the following equation. KBr (g) = V (1.063-D) / 0.68323

The half volume of the blood plasma fraction containing LDL that had been adjusted to achieve "d = 1.063" was dispensed into 6 centrifuge tubes. A density solution (d = 1.063) was introduced thereinto to the level of the rim of each centrifuge tube. Ultracentrifugation was performed at 100,000 x g (36,000 rpm) at 4°C for 20 hours with the use of an RP-42 Rotor. Then, the top layer (yellow layer) (crude LDL) was recovered with a Pasteur pipette. The remaining half volume of the above fraction was dispensed in the same manner, followed by ultracentrifugation. Then, the top layer (yellow layer) (crude LDL) was recovered. The separate portions of crude LDL obtained by the two above operations were mixed together. The resultant was dispensed into 6 centrifuge tubes. A density solution (d = 1.063) was introduced thereinto to the rim of each centrifuge tube. Ultracentrifugation was performed at 100,000 x g (36,000 rpm) at 4°C for 20 hours with the use of an RP-42 Rotor. Then, the top layer (yellow layer) (purified LDL) was recovered. LDL was added to a dialysis membrane, followed by dialysis with the use of EDTA-Saline (4 L) in a room at a low temperature. 12-hour dialysis was repeated 3 times. The resultant was concentrated using ULTRAFILTRATION CELL MODEL 8200 (AMICON) such that the protein concentration became approximately 2 mg/mL, followed by filtration sterilization using a filter. The protein concentration was determined by the BCA method (Pierce). LDL was used for preparation of AcLDL described below.

AcLDL was prepared in the manner described below.

Equivalent volumes of LDL (protein: 30 mg) (15 mL if 2.0 mg protein/mL) and saturated sodium acetate were mixed together. The mixture was cooled on ice and stirred with a stirrer bar, during which acetic anhydride (150 µL) (5 µL/LDL mg protein) was added dropwise (5 µL each) [Miyazaki A et al., J Biol Chem. 1994;269:5264-5269.]. At this time, pH was monitored with a pH meter with the dropwise addition of 5 N NaOH thereto such that the pH could be maintained at more than 6.5. The resultant was allowed to stand on ice for 1 hour, followed by dialysis with the use of EDTA-Saline (4 L) in a room at a low temperature. 12-hour dialysis was repeated 3 times. The resultant was concentrated using ULTRAFILTRATION CELL MODEL 8200 (AMICON). The protein concentration was determined by the BCA method (Pierce). The thus obtained AcLDL was used for experiments.

The reagents used for the operations described below are as follows.
[9,10(n)-³H] oleic acid (185 MBq in 1 mL toluene, GE Healthcare Bio-Science) (oleic acid mole concentration: 0.625 mM)
3 mM Oleic Acid-Albumin from bovine serum (SIGMA): The albumin concentration was 100 mg/mL (1.5 mM) with the binding of 2-mol oleic acid in 1 mol of the reagent.

### Preparation of 3 mM [³H]oleic acid mixture

[9,10(n)-³H] oleic acid (50 µL) (9.25 MBq) was introduced into a glass beaker and dried with nitrogen. 3 mM Oleic Acid-Albumin from bovine serum (SIGMA) (5 mL) was poured into the glass beaker, following stirring with a stirrer bar for 5 hours, followed by filtration sterilization with a 2-µm filter.

Human peripheral blood monocytes (4 × 10⁶/6 cm dish) were cultured for 7 days in an RPMI-1640 medium (2 mL) containing 10% human serum so as to differentiate into macrophages. At such time, 0.6 nM salusin-α or salusin-β was added during culture. Effects of salusin-α and salusin-β on intracellular cholesterol ester accumulation (cholesterol esterification) were examined. On Day 7, the medium was changed. 5 µg/mL AcLDL, a 3 mM [³H]oleic acid mixture (67 µL) (final concentration: 0.1 mM), and 0.6 nM salusin-α or salusin-β were added to the cells. An adequate amount of a medium was further added thereto so as to result in a total amount of 2 mL, followed by another instance of culture for 18 hours. The cells were washed 3 times with PBS. Hexane/isopropanol (3:2, v/v) (1 mL) was added to each dish. Each dish was allowed to stand at room temperature for 1 hour. Hexane/isopropanol was recovered into a lipid extraction tube. Further, 1 mL of hexane/isopropanol was added to each dish for washing the cells and the dish walls and then recovered into the same lipid extraction tube. After lipid extraction, 1 N NaOH (1 mL) was added to each dish for protein extraction. The lipid extraction tube was vortexed and allowed to stand at room temperature for 30 minutes, followed by drying with nitrogen. The extracted cell lipid was vortexed and dissolved with the addition of 60 µL isopropanol. The total amount thereof was spotted between standard spots in a broad manner, Development was carried out by TLC in a manner such that the development solvent (hexane /diethylether /acetic acid (90 : 10 : 1, v/v)) reached the top edge of the TLC plate. The TLC plate was placed in a glass chamber for color development of lipids with iodine. The portion corresponding to cholesterol oleate was excised with scissors and such portion was placed in a scintillation vial, followed by assay with the addition of a scintillation cocktail (Perkin Elmer) (3 mL). IN NaOH that had been previously added to each dish was recovered and the cell protein was quantitatively assayed by the BCA method (Pierce). The specific activity (dpm/nmol) of a 3 mM [³H]oleic acid mixture was assayed. The cholesterol esterification (nmol/mg cell protein) was calculated by dividing the radioactivity (dpm) of cholesterol [³H]oleate produced in the cells by the specific activity (dpm/nmol) and the cell protein amount (mg) of [³H]oleic acid.

ACAT-1 is an important factor of promoting foaming of macrophages. The above experiments revealed that salusin-α and salusin-β influence ACAT-1 expression and ACAT activity in a conflicting manner. Accordingly, the effects of salusin-α and salusin-β on foaming of macrophages were examined.

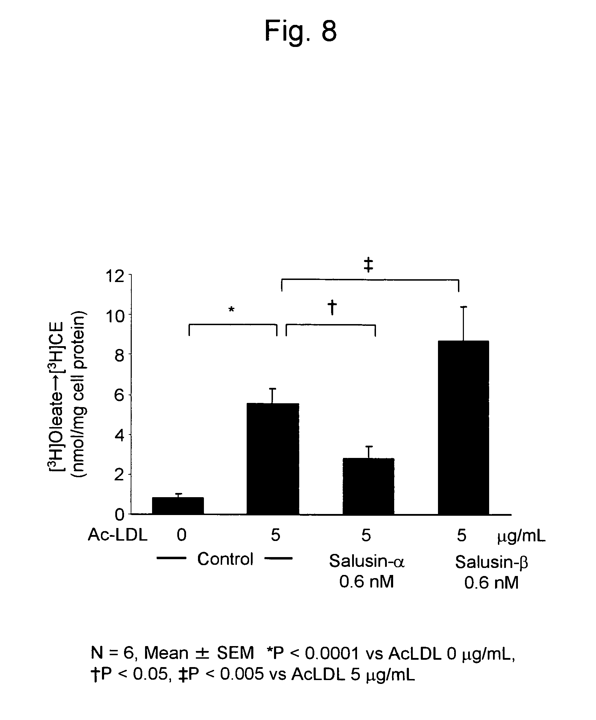

Human peripheral blood monocytes (4 × 10⁶ cells/6 cm dish) were cultured for 7 days so as to differentiate into macrophages. At such time, effects of 0.6 nM salusin-α and salusin-β, which had been simultaneously added during culture, on foaming of macrophages were examined. 5 µg/mL AcLDL, a 0.1 mM [³H]oleic acid mixture, and 0.6 nM salusin-α or salusin-β were added to the cells on Day 7 of culture. The cells were further cultured for 24 hours, followed by assay of cholesterol [³H]oleate accumulated in the cells. When control cells were loaded with AcLDL, accumulation of cholesterol [³H]oleate was observed at 5.6 nmol/mg cell protein. The result was 6.3 times as great as that obtained for the cells loaded with AcLDL (non-loaded control cells). The degree of accumulation of cholesterol [³H]oleate caused by AcLDL in the cells treated with salusin-α decreased to a level corresponding to 50% of that in the control cells (loaded control). The same in the cells treated with salusin-β increased to a level 1.6 times as great as that in the control cells (Fig. 8).

### Example 5: Examination of cell-incorporation capacity of acetylated LDL (AcLDL) - Determination of scavenger receptor class A (SR-A) activity

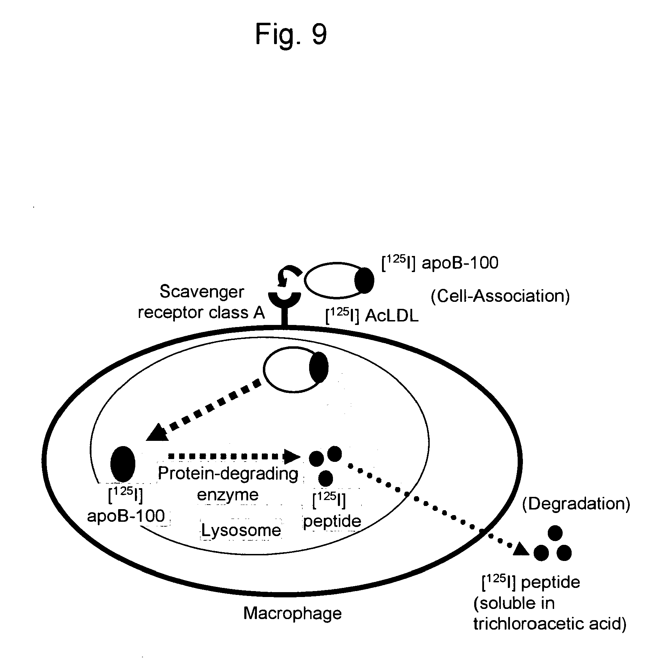

A scavenger receptor is a lipoprotein receptor expressed on macrophages. It recognizes modified LDL such as oxidized LDL or AcLDL, leading to endocytosis. A ligand of such receptor that has been incorporated into a cell is degraded in the lysosome. Such scavenger receptor plays a very important role on formation of foam cells from macrophages. Known examples of a scavenger receptor include a scavenger receptor class A (SR-A), CD36, lectin-like oxidized LDL receptor-1 (LOX-1), and CD68 [Greaves DR et al., J Lipid Res. 2005; 46: 11-20.]. Oxidized LDL can bind to the four types of the above scavenger receptors. However, the only scavenger receptor to which AcLDL binds is SR-A. In this experiment, AcLDL, which is a representative example of modified LDL, was used as a ligand and the activity of SR-A serving an exclusive scavenger receptor capable of recognizing AcLDL was assayed (Fig. 9).

Peripheral blood human monocytes (4 × 10⁶cells/6 cm dish) were cultured for 7 days in an RPMI-1640 medium (2 mL) containing 10% human serum so as to differentiate into macrophages. At such time, 0.6 nM salusin-α or salusin-β was added during culture to examine effects of salusin-α and salusin-β on the endocytosis activity (SR-A activity) of [¹²⁵I]AcLDL. On Day 7, the medium was changed. An adequate amount of medium was added to [¹²⁵I]AcLDL (5 to 10 µg/mL) and 0.6 nM salusin-α or salusin-β so as to result in a total amount of 2 mL. The cells were further cultured for 18 hours.

The cells were washed once with PBS containing 3% BSA and then washed twice with PBS. 0.1 N NaOH (1 mL) was added thereto and the resultant was allowed to stand at room temperature for 30 minutes. The cells were recovered into a polystyrene tube (12 x 75 mm, Fisher Scientific Company), further washed with 0.1N NaOH (1 mL), and recovered again. The total amount of the cell extraction solution was assayed using a γ counter. The protein concentration of a portion thereof was determined by the BCA method (Pierce). The cell association (µg/mg cell protein) of [¹²⁵I]AcLDL was calculated by dividing the radioactivity (cpm) of the cell extraction solution by the specific activity (cpm/µg) of [¹²⁵I]AcLDL and the cell protein amount (mg) per dish [Miyazaki A et al., J Biol Chem. 1994; 269: 5264-5269].

Degradation assay was carried out by the method described below.

[¹²⁵I]AcLDL incorporated by macrophages via scavenger receptors are degraded in the lysosome. [¹²⁵I] apo B-100, which is a protein portion of [¹²⁵I]AcLDL, is fragmented by a protein-degrading enzyme to be formed into peptides so as to be extracellularly excreted, and it then can be found in a trichloroacetic acid soluble fraction. Meanwhile, [¹²⁵I]AcLDL that has not been incorporated by macrophages in a medium is insoluble in trichloroacetic acid and thus can be separated in the form of a precipitate by centrifugation.

A portion (750 µL) was collected from each culture supernatant into a polystyrene tube (12 x 75 mm, Fisher Scientific Company). 40% trichloroacetic acid (250 µL) and 0.7 M AgNO₃ (200 µL) were added thereto and the mixture was vortexed, followed by centrifugation at 2,500 rpm (TOMY, LC-120low-speed centrifuge, rotor 7015-06) for 10 minutes. The major portion of free ¹²⁵I that had not reacted with protein in the step of labeling AcLDL with iodine was removed by gel filtration. However, a portion thereof was found to remain. In this step, the remaining portion of ¹²⁵I could be precipitated in the form of Ag[¹²⁵I] with the addition of AgNO₃. Therefore, the trichloroacetic acid insoluble fraction of the culture supernatant contained [¹²⁵I]AcLDL that had not been incorporated by cells and Ag[¹²⁵I] derived from free ¹²⁵I. In addition, the peptide from [¹²⁵I]AcLDL that had been processed by cells was exclusively detected in the trichloroacetic acid soluble fraction. After centrifugation, the supernatant (600 µL) was collected into a different tube and assayed with a γ counter. Based on the assay results, the total radioactivity (cpm) of the trichloroacetic acid soluble fraction in the culture supernatant was calculated for each dish. The [¹²⁵I]AcLDL degradation (µg/mg cell protein) was obtained by dividing the total radioactivity by the specific activity (cpm/µg AcLDL protein) of [¹²⁵I]AcLDL and the cell protein amount (mg) per dish.

As a result of the above experiments, it was revealed that salusin-α and salusin-β control foaming of macrophages. Therefore, it was examined whether the SR-A activity controlled by salusin would influence the above phenomenon. The protein portion (apo B-100) of intracellularly incorporated AcLDL was degraded into peptides by a protein-degrading enzyme in the lysosome so as to be extracellularly excreted. Upon association assay, the radioactivity of ¹²⁵I existing on the cell surfaces or inside cells was assayed. Upon degradation assay, the radioactivity of [¹²⁵I]AcLDL (fragment peptide of apo B-100) that had been fragmented into peptides in the lysosome and extracellularly secreted was assayed. Based on the two assays, the SR-A activity was determined.

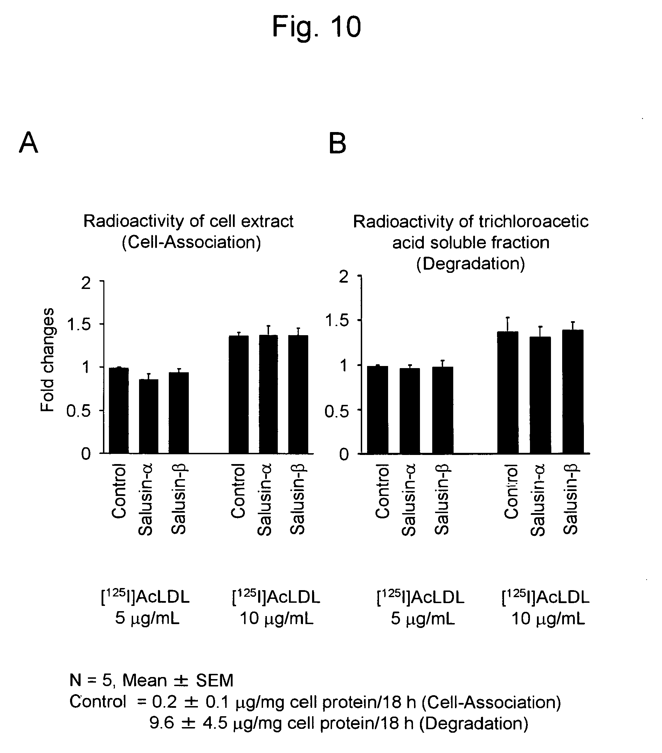

Human peripheral blood monocytes (4 × 10⁶ cells/6 cm dish) were cultured for 7 days so as to differentiate into macrophages. At such time, 0.6 nM salusin-α or salusin-β was added during culture. Then, effects of salusin-α and salusin-β on incorporation and degradation of [¹²⁵I]AcLDL were examined. Herein, 5 to 10 µg/mL [¹²⁵I]AcLDL and 0.6 nM salusin-α or salusin-β were added to the cells on Day 7 of culture and the cells were further cultured for 18 hours. Then, association assay and degradation assay were carried out. As a result, no significant changes caused by salusin-α and salusin-β were observed in either assay compared with control cells. (Figs. 10A and 10B and Fig. 10A show association assay results and Fig. 10B shows degradation assay results.) Accordingly, it has been revealed that salusin-α and salusin-β influence the metabolism of cholesterol after incorporation of AcLDL into cells but not the SR-A activity.

### Example 6: Effects of salusin-α and salusin-β on ATP-binding cassette transporter A1(ABCA-1) protein expression in human monocyte-derived macrophages

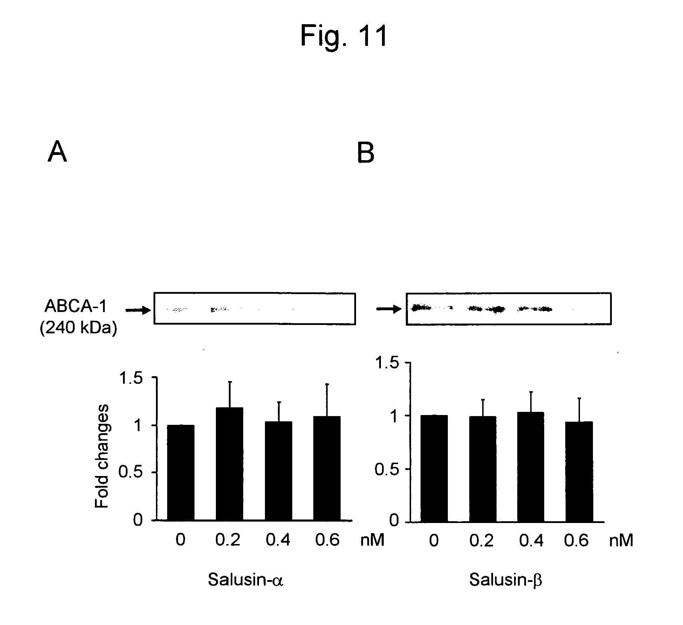

One mechanism of controlling the phenomenon of foaming of macrophages is the mechanism of extracellular secretion of free cholesterol via ABCA-1 (cholesterol efflux). Effects of salusin on the ABCA-1 protein expression were examined. Peripheral blood human monocytes (4 × 10⁶ cells /6 cm dish) were cultured for 7 days so as to differentiate into macrophages. At such time, salusin-α or salusin-β (0, 0.2, 0.4, 0.6, 0.8, or 1.0 nM) was added during culture. Then, effects of salusin-α and salusin-β on ABCA-1 protein expression were examined. In the aforementioned case of Western blotting with the use of an ABCA-1-specific antibody, salusin-α and salusin-β were found to have caused no significant changes in the ABCA-1 protein expression. (Figs. 11A and 11B: Fig. 11A shows the results for salusin-α and Fig. 11B shows the results for salusin-β.)

### Example 7: Effects of salusin-α and salusin-β on ACAT-1 protein expression in cultured human monocytes-derived macrophages after differentiation

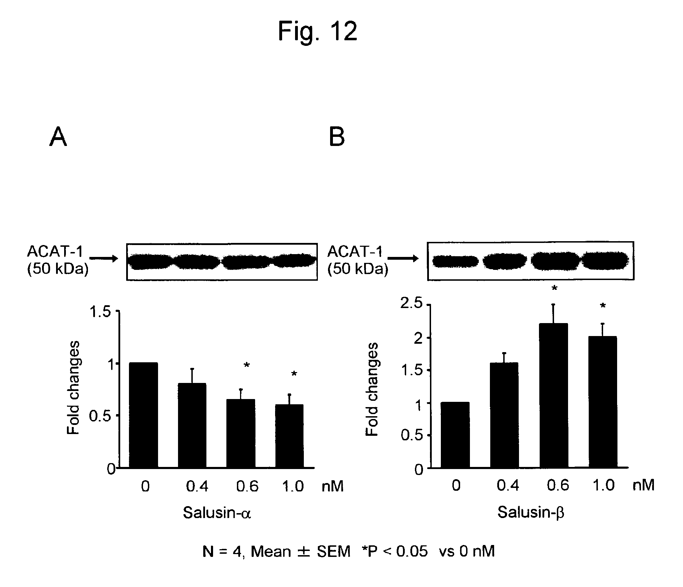

In each of the experiments in Examples 1 to 6, salusin-α and salusin-β were added in the phase of differentiation of cultured human monocytes into macrophages and cells obtained on Day 7 of culture were used to examine the effects of salusin-α and salusin-β. In this Example, it was examined whether salusin-α and salusin-β would have similar effects on ACAT-1 protein expression in macrophages obtained after differentiation. Peripheral blood human monocytes (4 × 10⁶ cells /6 cm dish) were cultured for 7 days so as to differentiate into macrophages. On Day 7, salusin-α and salusin-β (0, 0.4, 0.6, or 1.0 nM) were added thereto and the macrophages were further cultured for 3 days. As a result of examination by Western blotting of ACAT-1, the ACAT-1 protein expression level was decreased by 1.0 nM salusin-α to a level corresponding to 60% of that of the control. Meanwhile, the same was increased by 0.6 nMb salusin-β to a level 2.2 times as great as that of the control (Figs. 12A and 12B and Fig. 12A show the results for salusin-α and Fig. 12B shows the results for salusin-β.). Based on the results, it has been revealed that the control of the ACAT-1 expression by salusin-α or salusin-β is maintained even after differentiation of monocytes into macrophages.

The following are the main results obtained in the above Examples by examining effects of novel vasoactive materials, namely, salusin-α and salusin-β, on foaming of cultured human monocyte-derived macrophages (intracellular cholesterol ester accumulation) and the gene expression involved in such foaming.
(1) Foaming of macrophages caused by AcLDL was suppressed by salusin-α and promoted by salusin-β.
(2) The expression of the intracellular cholesterol esterification enzyme ACAT-1 was suppressed by salusin-α and promoted by salusin-β.
(3) The activity of SR-A capable of recognizing and intracellularly incorporating AcLDL was not influenced by salusin-α or salusin-β.
(4) The expression of ABCA-1, which plays an important role in transportation of intracellular cholesterol, was not influenced by salusin-α or salusin-β.

Based on the above, it has been revealed that foaming of cultured human monocyte-derived macrophages is controlled by salusin-α and salusin-β in a conflicting manner, and that a mechanism of controlling such foaming involves control of ACAT-1 expression by salusin-α and salusin-β in a conflicting manner.

Also, it has been reported that positive effects of salusin-β on promotion of proliferation of vascular smooth muscle cells or fibroblasts are not suppressed by pretreatment with salusin-α and therefore additive effects of salusin-α and salusin-β can be obtained [Shichiri M et al., Nat Med. 2003; 9: 1166-1172]. The report suggests that salusin-α and salusin-β probably act on such cells via different receptors. In addition, Wang et al. have reported that salusin-β is regarded as a ligand in HEK293T cells in which a mouse mas-like G protein-coupled receptor A1 (mMrgA1) has been expressed [Wang Z et al., Eur J Pharmacol. 2006; 539: 145-150]. Meanwhile, salusin-α does not serve as a ligand for mMrgA1, suggesting that salusin-α and salusin-β are recognized by different receptors [Wang Z et al., Eur J Pharmacol. 2006; 539: 145-150]. In this study, the obtained results indicate that salusin-α and salusin-β act on foaming of macrophages and ACAT-1 expression in a conflicting manner. Therefore, it can be assumed that salusin-α and salusin-β acted via different receptors expressed in macrophages.

### Example 8: Expression of salusin-α and salusin-β in arteriosclerotic lesions of human coronary arteries

Pathological coronary artery sections were prepared from a patient who had died of acute coronary syndromes (ACS) (72-year-old male) and stained with anti-salusin-α and -β antibodies that had been purified for immunochemical tissue staining. Strong salusin-β expression was observed not only in smooth muscle cells and fibroblasts in the plaque portions of the coronary artery but also in macrophage-derived foam cells in fatty streaks (Figs. 13A to 13D). Salusin-α and salusin-β are thought to be produced with a common portion of a common precursor (Preprosalusin). However, substantially no salusin-α expression was observed in the arteriosclerotic lesions of the coronary artery in which salusin-β had been strongly expressed. Similar findings were confirmed also in 3 other patients (a 40-year-old male, a 76-year-old female, and a 86-year-old female). As a result of the above examination, it has been demonstrated that the strong salusin-β expression and decreased salusin-α expression are deeply involved in the progress of human coronary arteriosclerosis.

### Example 9: Detection of arteriosclerosis with the use of salusin-α as a marker

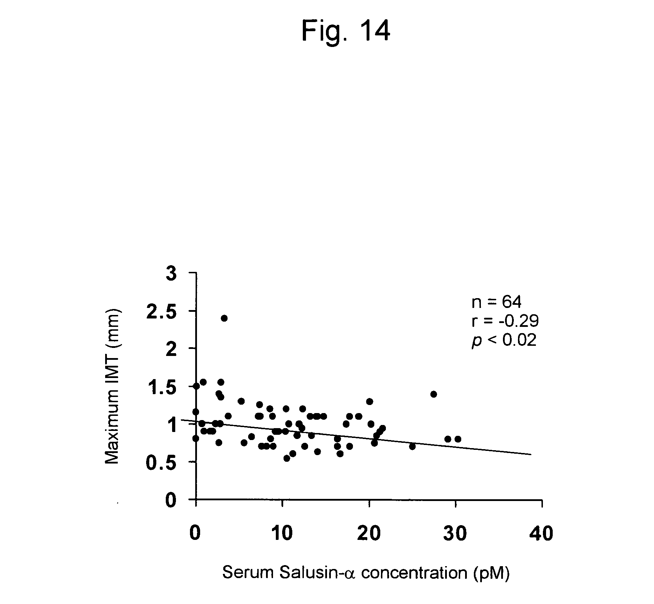

The relationship between serum salusin-α concentration and arteriosclerosis was examined for 64 middle-aged-to-elderly males (aged 40 to 86). For arteriosclerosis evaluation, carotid artery echocardiography (mode B), which can be performed in a simple and non-invasive manner, was used. The maximum level (maximum IMT) of intima-media complex thickness (IMT) of the carotid artery on each side was determined. The maximum IMT is 1 mm or less in healthy persons. It has already been demonstrated that a maximum IMT of more than 1 mm is a risk factor of acute coronary syndromes (ACS), stroke, and the like. The serum salusin-α concentration was assayed by radioimmunoassay described in Sato K et al., Peptides. 2006; 27: 2561-2566. There was a significant negative correlation between the serum salusin-α concentration and the maximum IMT of the carotid artery (r = 0.29, P < 0.02; Fig. 14). This indicates that the lower the serum salusin-α concentration, the more advanced the stage of arteriosclerosis.

### Example 10: Detection of acute coronary syndromes, ischemic heart disease, and arteriosclerosis with the use of salusin-α as a marker

Serum samples were collected from the following cases: ischemic heart disease cases composed of 37 stable exertional angina pectoris patients (26 male cases and 11 female cases), 60 acute coronary syndrome patients (within 6 hours after onset) (41 male cases and 19 female cases), and 76 patients with old myocardial infarction (after 6 months or more had passed since the onset of acute coronary syndromes) (61 male cases and 15 female cases) (173 cases in total); and control cases composed of 40 essential hypertension patients (28 male cases and 12 female cases) and 55 healthy volunteers (35 male cases and 20 female cases). Salusin-α concentration was assayed by radioimmunoassay according to the descriptions in Sato K et al., Peptides. 2006; 27: 2561-2566. All of the cases of acute coronary syndromes were subjected to emergency coronary artery imaging. The severity of coronary artery lesion was evaluated based on the number of coronary artery branches found to have arteriosclerotic lesions (right coronary artery + left anterior descending artery + left circumflex artery = 3 branches in total). In addition, all of the hypertension cases and 23 healthy volunteers were subjected to carotid artery echocardiography. A lesion with an IMT of 1.1 mm or more with a clear boundary was designated to be a plaque lesion. The plaque score was determined as the total sum of the thicknesses of all plaque lesions (right and left). A plaque score of 1.1 to 5 was designated as corresponding to light arteriosclerosis. A plaque score of 5 to 10 was designated as corresponding to moderate arteriosclerosis, and a plaque score of more than 10 was designated as corresponding to severe arteriosclerosis.

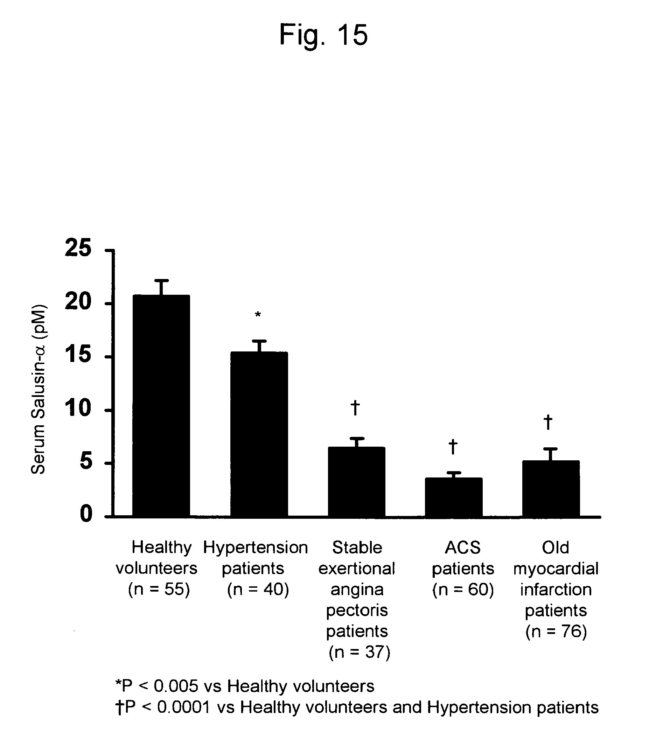

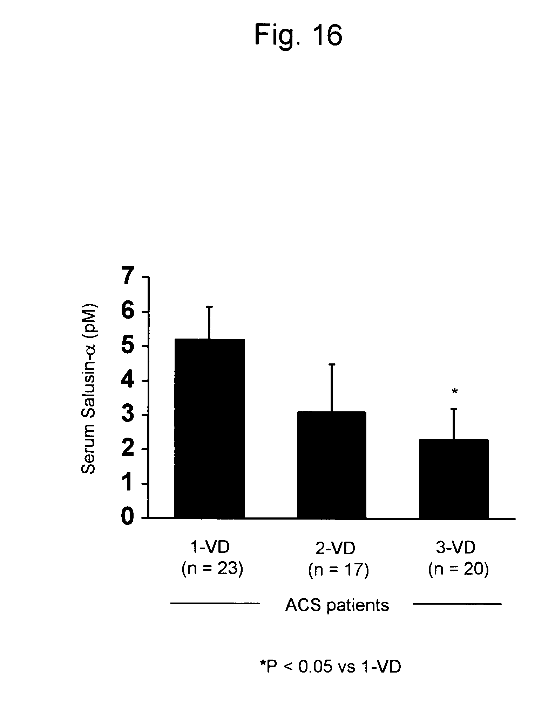

The serum salusin-α concentration was 4.9 ± 0.6 pM for the ischemic heart disease patients. The figure was significantly lower than that for healthy volunteers (21.7 ± 1.5 pM) (Mean ± SEM, hereinafter same as above) and that for hypertension patients (15.4 ± 1.1 pM) (p < 0.0001). Among the ischemic heart disease patients, the serum salusin-α concentration was stable for the exertional angina pectoris patients (6.4 ± 0.9 pM), the same was 3.6 ± 0.6 pM for the patients with acute coronary syndromes, and the same was 5.2 ± 1.2 pM for the patients with old myocardial infarction (Fig. 15). As a result of emergency coronary artery imaging of 60 acute coronary syndrome patients, 23 cases thereof were found to have the 1-vessel disease, 17 cases thereof were found to have the 2-vessel disease, and 20 cases thereof were found to have the 3-vessel disease. The salusin-α concentration was 5.2 ± 1.0 pM for the 1-vessel disease patients, the same was 3.1 ± 1.4 pM for the 2-vessel disease patients, and the same was 2.3 ± 0.9 pM for the 3-vessel disease patients. The results for the 3-vessel disease patients were significantly lower than those for the 1-vessel disease patients (p < 0.05, Fig. 16).

There was a negative correlation between the maximum IMT of the carotid artery and the salusin-α concentrations for 40 hypertension patients and 23 healthy volunteers. In addition, the plaque score gradually decreased as the severity increased. Accordingly, it is thought that reduction in salusin-α concentration is involved in the progress of arteriosclerosis.

In view of the above, it is assumed that reduction in salusin-α concentration reflects the presence of arteriosclerosis, and in particular, ischemic heart disease. For acute coronary syndromes, a lower salusin-α concentration was obtained than that obtained in the case of any other ischemic heart disease. In addition, it was found that the salusin-α concentration further decreased as the severity of coronary artery lesion increased. This revealed that salusin-α, which has the anti-arteriosclerosis action, is highly useful as a marker indicating the onset or the severity of an acute coronary syndrome.

### Example 11: Effects of continuous administration of salusin-α and salusin-β on arteriosclerosis

Effects of continuous administration of salusin-α and salusin-β on arteriosclerosis were examined with the use of apoE-deficient mice as arteriosclerosis animal models. Herein, antiserum effects of salusin-α or salusin-β were also examined. In addition to examination of arteriosclerotic lesions, foaming of macrophages, which is important for formation of arteriosclerosis primary lesions, was focused on and cholesterol ester accumulation caused by oxidized LDL was assayed. Expression analysis was carried out for scavenger receptors (CD36 and SR-A) capable of recognizing oxidized LDL, an intracellular cholesterol esterification enzyme (acyl-CoA) (cholesterol acyltransferase 1 (ACAT1)), and ATP-binding cassette transporter A1 (ABCA1), ABCG1, and SR-BI, which are involved in the mechanism of extracellular transportation of excessive intracellular free cholesterol (cholesterol efflux).

The above animal experiments were carried out in accordance with the experimental practice guidelines established by Showa University (Japan). All the apoE-deficient mice used for this study were purchased from the Jackson Laboratory (Bar Harbor, ME, U.S.A.).

13-week-old male apoE-deficient mice (72 mice) were divided into the following 6 groups: (i) physiological salt saline (control); (ii) salusin-α (0.6 nmol/kg/h); (iii) salusin-β (0.6 nmol/kg/h); (iv) salusin-α (0.6 nmol/kg/h) + anti-salusin-α serum (1.4 µg/kg/h); (v) salusin-β (0.6 nmol/kg/h) + anti-salusin-β serum (1.4 µg/kg/h); and (vi) anti-salusin-β serum (1.4 µg/kg/h). The mice were subjected to continuous subcutaneous administration with the use of an osmotic mini-pump for 4 to 8 weeks. The osmotic mini-pump and the solution contained therein were changed every week.

Mice ingesting a normal diet were subjected to experiments. The following parameters were assayed.

### 1. Analysis of arteriosclerotic lesions of aorta

Each collected aorta was cut in the longitudinal direction, followed by oil red O staining for examination of the surface area of each atherosclerosis lesion with lipid pigmentation. Adobe Photoshop (Adobe System Inc., San Jose, CA, U.S.A.) was used for identification of arteriosclerotic lesions, followed by analysis using NIH Scion Image (Scion Corp., Frederick, MD, U.S.A.) for quantification of the surface area of each lesion. In addition, cross-sections of the vicinity of the aorta (immediately below the aortic valve) were prepared, followed by oil red O staining for detailed analysis of arteriosclerotic lesions.

### 2. Analysis of macrophages

### (1) Examination of intracellular cholesterol ester accumulation

On Day 4 after intraperitoneal administration of thioglycollate, transudatory macrophages were collected and cultured with an RPMI1640 solution containing 10% bovine fetal serum. Oxidized LDL (10 µg/mL) and [³H] oleic acid (0.1 mM) were added to the culture solution. After 13 hours, the radioactivity of intracellularly accumulated cholesterol [³H] oleic acid was assayed for evaluation of foaming of macrophages.

### (2) Examination of cholesterol efflux

Macrophages collected in the above manner were kept warm for 24 hours in a culture solution prepared as above containing oxidized LDL (10 µg/ml) labeled with [³H]cholesterol (74 kBq/mL) and washed twice with PBS. Thereafter, the macrophages were kept warm for 16 hours in an RPMI1640 solution containing 0.1% BSA to which HDL (15 µg/mL) had been added. The culture solution was centrifuged at 15,000 rpm for 10 minutes so as to remove the residues therefrom. The cells were washed twice with PBS and then dissolved in 1N NaOH (0.3 mL). The radioactivity of [³H]cholesterol in the culture solution and that in the cell extraction solution were assayed: cholesterol efflux (%) = the radioactivity of [³H]cholesterol in the culture solution / the radioactivity of [³H]cholesterol in the culture solution + the radioactivity of [³H]cholesterol in the cell extraction solution × 100

### (3) Examination of expression of the gene involved in foaming

Macrophages were collected as in the experiment in (1), and the protein expression of the gene involved in foaming of macrophages (i.e., scavenger receptors CD36 and SR-A capable of recognizing oxidized LDL, intracellular cholesterol esterification enzyme ACAT1, and ABCA1, ABCG1, and SR-BI, which are involved in cholesterol efflux) was analyzed by Western blotting. For the internal standard of the macrophage function, β-action expression was also examined. For quantification of the band concentration, Analyzer (ATTO Corporation, Tokyo) was used.

As a result of the above examination, the following results were obtained.

### 1. Arteriosclerotic lesions of aorta

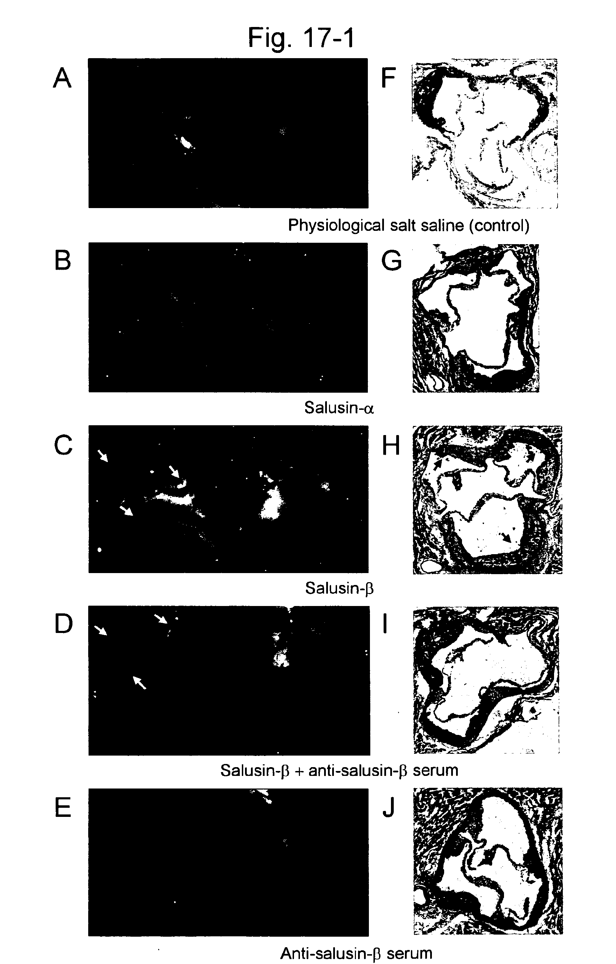

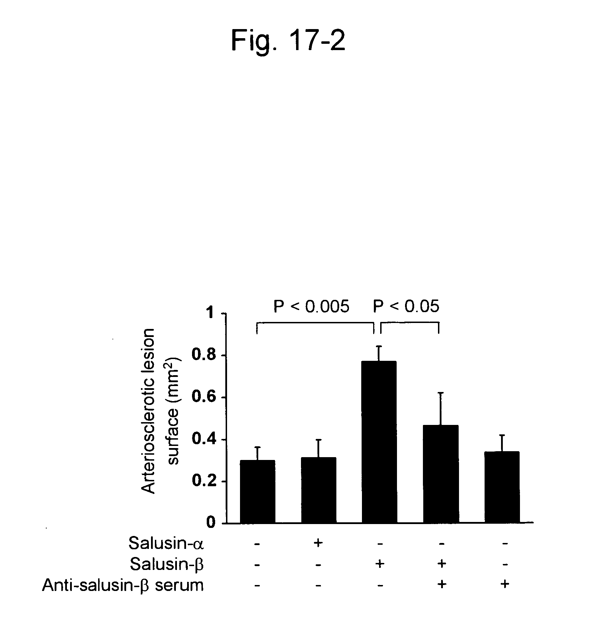

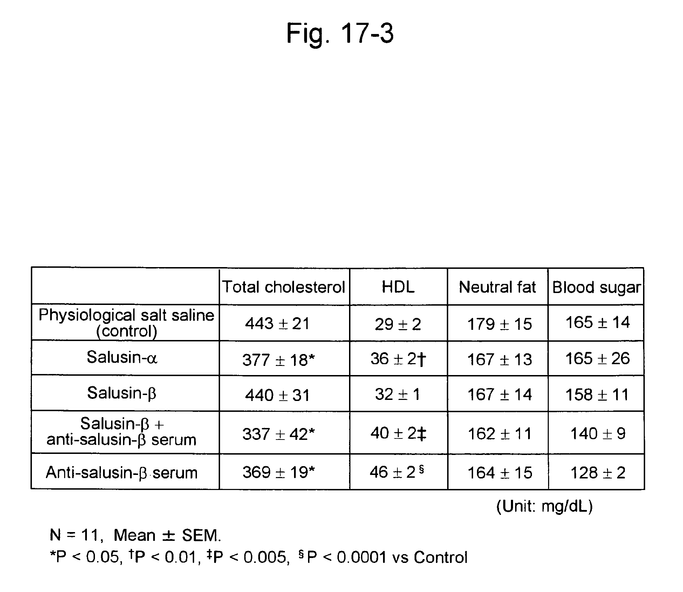

Four weeks after administration, arteriosclerotic lesions of the aortic arch stained red with oil red O were observed in a wide area ranging from the aortic root to the bifurcation of the brachiocephalic artery in apoE-deficient mice treated with salusin-β (Fig. 17-1C, arrow), compared with control mice treated with physiological salt saline (Fig. 17-1A). Regarding the mean value of arteriosclerotic lesion surface area assayed for each group (consisting of 6 mice), the mean value for the group treated with salusin-β was found to have increased to approximately 2.5 times that of the control group (P < 0.005, Fig. 17-2). In proximal aorta cross-sections, formation of atheroma stained red with oil red O and thickening of the blood vessel wall were observed in the group treated with salusin-β (Fig. 17-1H, arrow), compared with the control group (Fig. 17-1F). In mice treated with anti-salusin-β serum in addition to salusin-β, arteriosclerotic lesions of aorta (Fig. 17-1D), atheroma, and thickening of the blood vessel wall (Fig. 17-1I) were improved compared with mice treated with salusin-β alone. In the mice treated with anti-salusin-β serum alone, arteriosclerotic lesions similar to those of the control mice were observed (Figs. 17-1E and J).

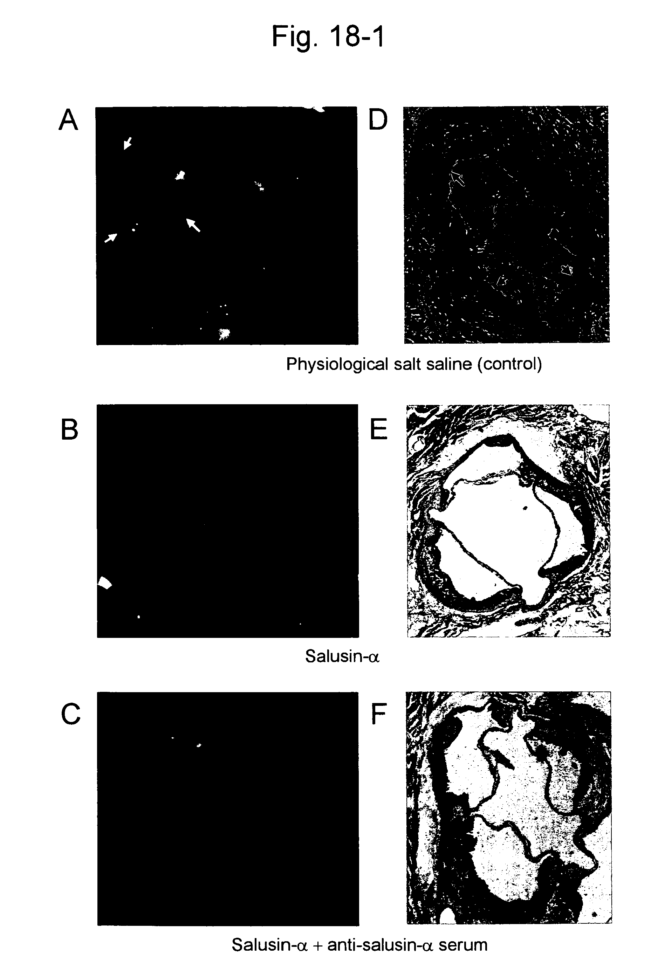

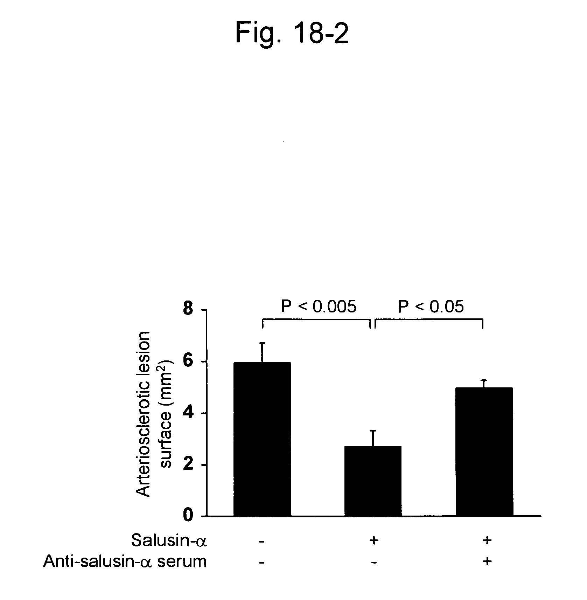

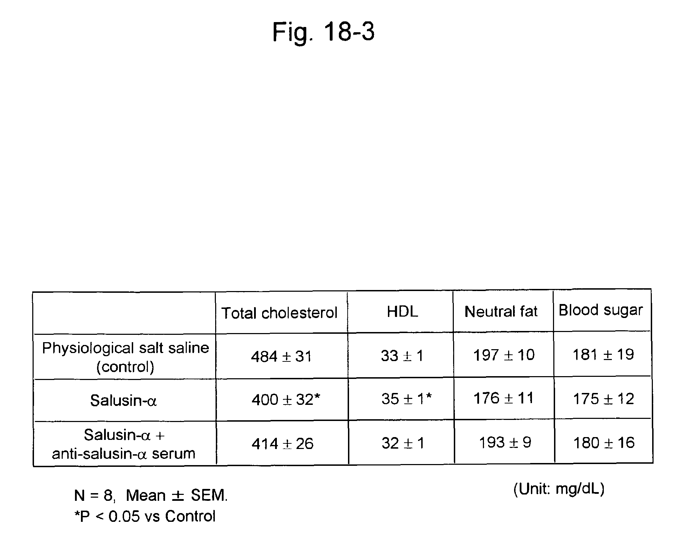

Eight weeks after administration, the size of the arteriosclerotic lesion in the aortic arch was significantly increased in the control mice treated with physiological salt saline (Fig. 18-1A, arrow). However, as a result of administration of salusin-α, the arteriosclerotic lesion surface area was reduced to less than half of such area before administration of salusin-α (Fig. 18-1B and 18-2). In addition, as a result of administration of salusin-α, obvious plaque formation was not observed (Fig. 18-1B and E). In the presence of anti-salusin-α serum, suppressive effects of salusin-α on arteriosclerotic lesions were cancelled (Fig. 18-1C and F and 18-2).

### 2. Analysis of macrophages

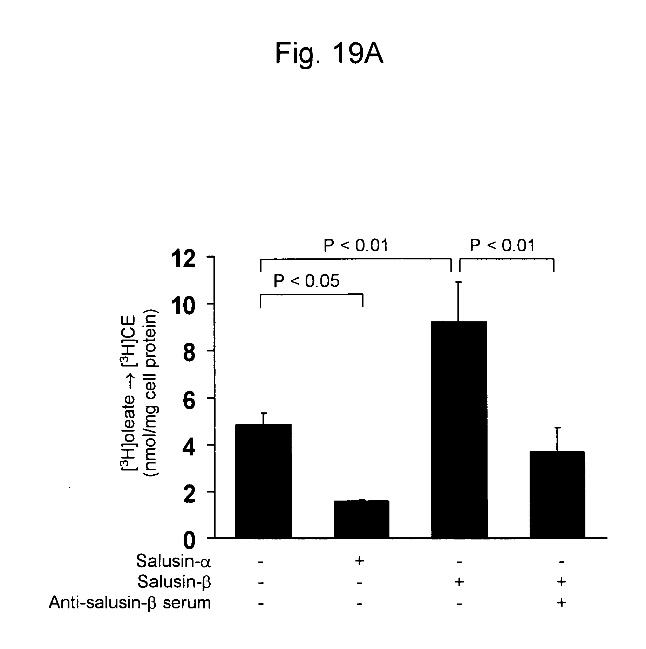

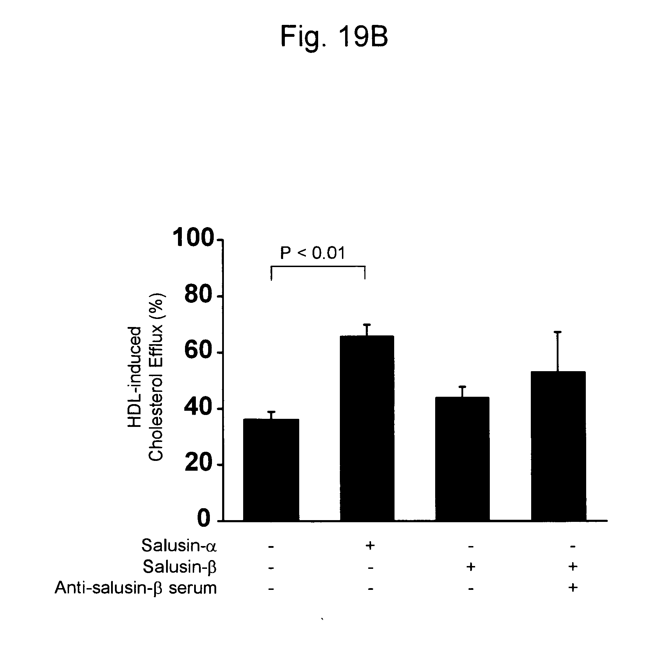

Four weeks after administration, in the case of administration of salusin-α, foaming of intraperitoneally collected transudatory macrophages due to oxidized LDL (intracellular cholesterol ester accumulation) was reduced to a level approximately 1/4 of that of the control (P < 0.05, Fig. 19A). In the case of administration of salusin-β, foaming of macrophages was increased to a level approximately 2 times that of the control (P < 0.01, Fig. 19A). However, it was suppressed to the control level in the presence of anti-salusin-β serum (P < 0.01, Fig. 19A). Cholesterol efflux caused by HDL was increased in the presence of salusin-α to a level approximately 2 times of that of the control (P < 0.01, Fig. 19B).

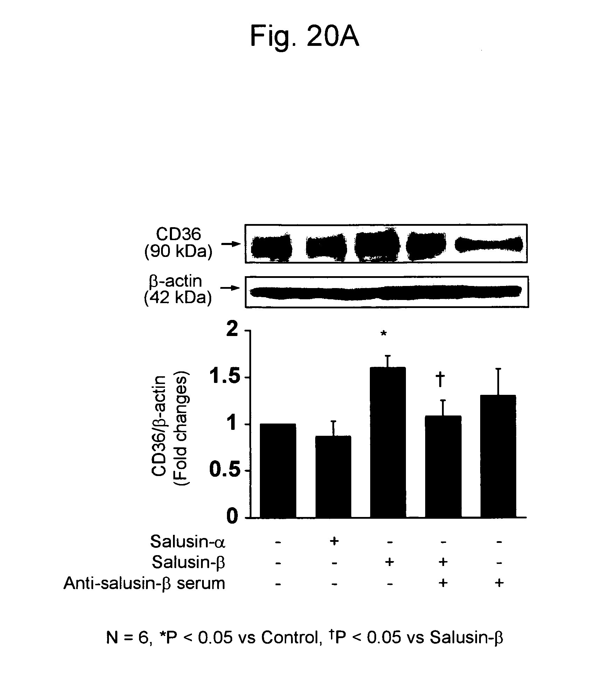

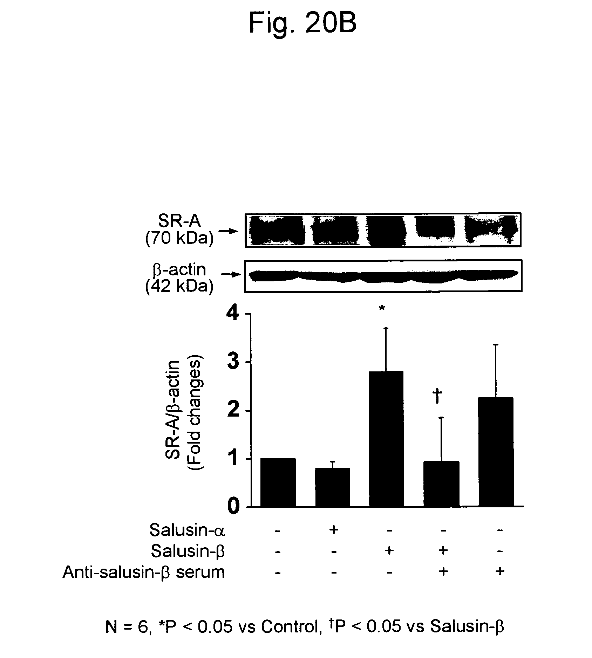

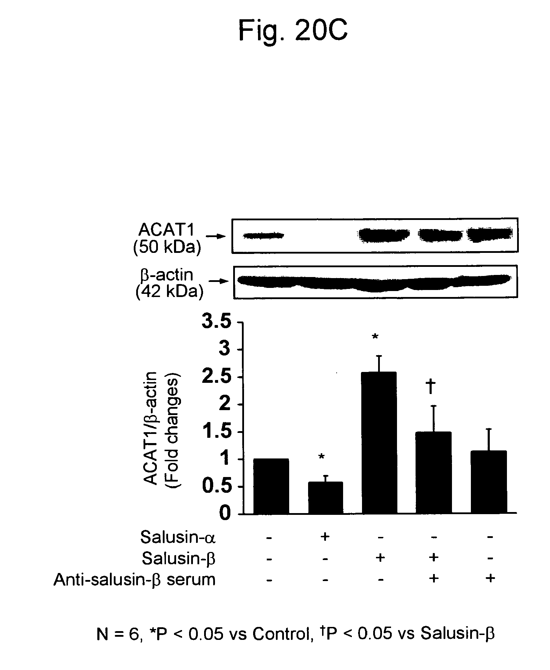

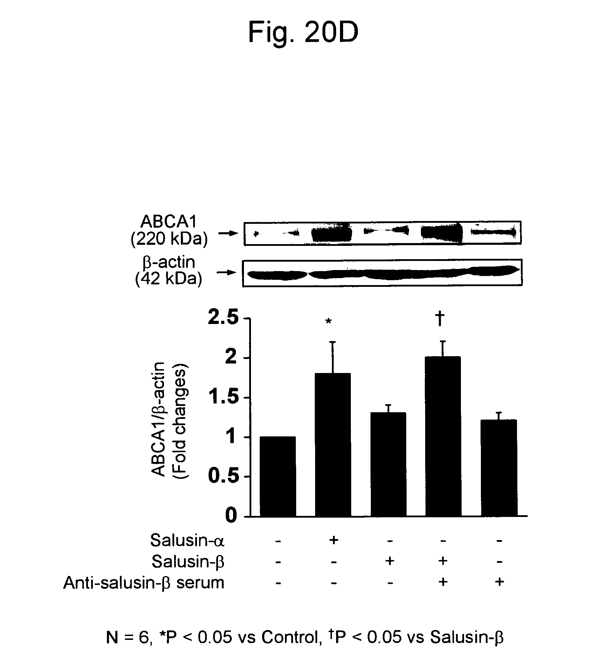

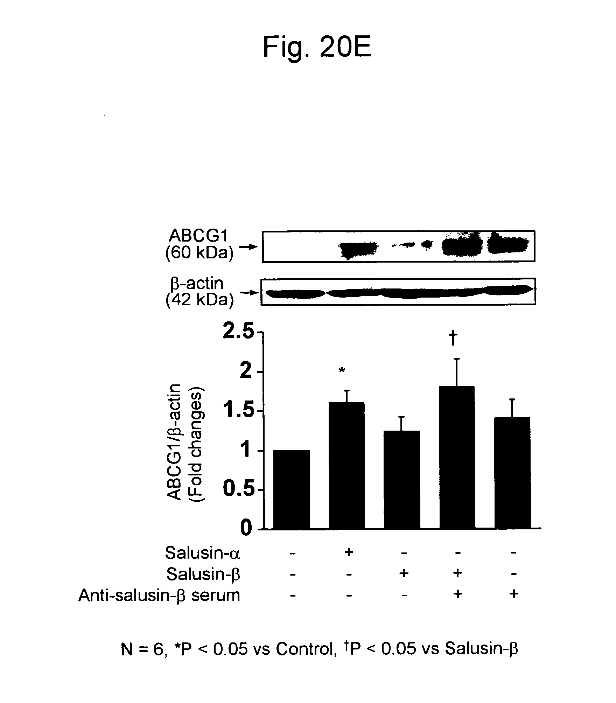

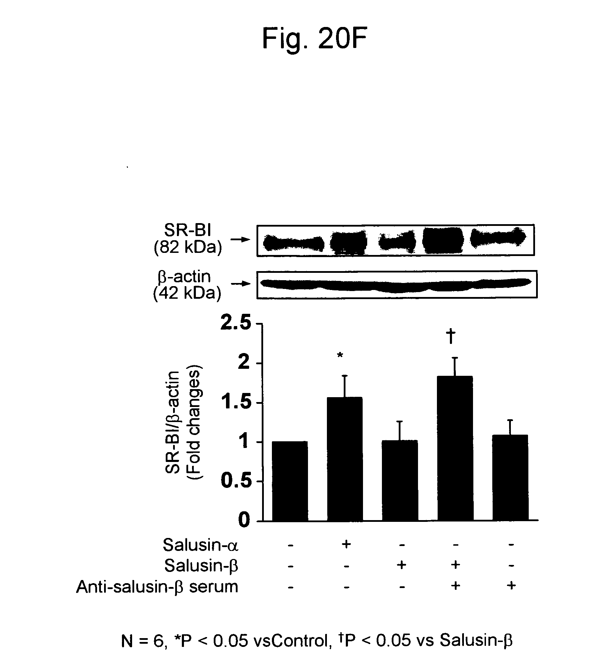

Fig. 20 shows the protein expression levels of the genes involved in foaming of macrophages at the same phase. Salusin-α did not influence the expression of scavenger receptors (CD36, SR-A) capable of recognizing oxidized LDL (Fig. 20A and B). However, salusin-α reduced the ACAT1 expression to a level that was approximately half of the initial level (P < 0.05, Fig. 20C) and promoted the expression of ABCA1, ABCG1, and SR-BI, which are involved in cholesterol efflux, to a level 1.5 to 2 times the initial level (P < 0.05, Fig. 20D, E and F). Meanwhile, salusin-β increased the expression of CD36, SR-A, and ACAT1 to a level approximately 1.5 to 2.8 times that of the control level (P < 0.05, Fig. 20A, B and C). The above effects of salusin-β were completely cancelled by anti-salusin β serum (Fig. 20A, B and C).

### Industrial Applicability

As described in the Examples, salusin-α suppresses foaming of macrophages so as to suppress arteriosclerotic lesion formation. Therefore, salusin-α can be used as a prophylactic therapeutic agent for arteriosclerotic diseases. Meanwhile, salusin-β promotes foaming of macrophages so as to promote arteriosclerotic lesion formation. Therefore, arteriosclerotic diseases can be prevented and treated by suppressing the action of salusin-β. The anti-salusin-β antibody suppresses the action of salusin-β and therefore it can be used as a prophylactic therapeutic agent for arteriosclerotic diseases. Further, the salusin-α concentration in a biological sample such as serum, urine, or the like relates to arteriosclerotic lesion formation. Therefore, salusin-α in a biological sample can be used as a marker for detection or risk evaluation of an arteriosclerotic disease.

An antagonistic compound that suppresses the action of salusin-α or salusin-β can be used for prevention/treatment of arteriosclerotic diseases. In addition, salusin-α can be used as a marker for detection of arteriosclerotic diseases. As described above, the present invention can be used in the field of medicine.

All publications, patents, and patent applications cited herein are incorporated herein by reference in their entirety.

## Claims

1. A therapeutic agent for an arteriosclerotic disease, comprising salusin-α as an active ingredient.

2. A therapeutic agent for an arteriosclerotic disease, comprising an antagonist of salusin-β as an active ingredient.

3. The therapeutic agent for an arteriosclerotic disease according to claim 2, wherein the antagonist of salusin-β is an anti-salusin-β antibody.

4. The therapeutic agent for an arteriosclerotic disease according to any one of claims 1 to 3, wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.

5. A therapeutic composition for an arteriosclerotic disease, comprising salusin-α and an antagonist of salusin-β.

6. The therapeutic composition for an arteriosclerotic disease according to claim 5, wherein the antagonist of salusin-β is an anti-salusin-β antibody.

7. The therapeutic composition for an arteriosclerotic disease according to claim 5 or 6, wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.

8. A method for detecting an arteriosclerotic disease, comprising assaying salusin-α in a biological sample.

9. The method for detecting an arteriosclerotic disease according to claim 8, wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.

10. The method for detecting an arteriosclerotic disease according to claim 8 or 9, wherein the biological sample is selected from the group consisting of serum, blood plasma, and urine.

11. A method for determining the severity of an arteriosclerotic disease, comprising assaying salusin-α in a biological sample.

12. The method for determining the severity of an arteriosclerotic disease according to claim 11, wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.

13. The method for determining the severity of an arteriosclerotic disease according to claim 11 or 12, wherein the biological sample is selected from the group consisting of serum, blood plasma, and urine.

14. A diagnostic marker for detecting an arteriosclerotic disease, comprising salusin-α.

15. The diagnostic marker for detecting an arteriosclerotic disease according to claim 14, wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.

16. A reagent for detecting an arteriosclerotic disease, comprising an anti-salusin-α antibody and using salusin-α as a diagnostic marker for detecting an arteriosclerotic disease.

17. The reagent for detecting an arteriosclerotic disease according to claim 16, wherein the arteriosclerotic disease is selected from the group consisting of acute coronary syndromes, an ischemic heart disease that is myocardial infarction or angina pectoris, brain infarction, encephalorrhagy, aortic aneurysm, aorta dissection, nephrosclerosis, and arteriosclerosis obliterans.
